(19) Europäisches Patentamt / European Patent Office / Office européen des brevets

(11) **EP 4 671 769 A1**

(12) **EUROPEAN PATENT APPLICATION**

(43) Date of publication:
31.12.2025 Bulletin 2026/01

(21) Application number: 25186460.9

(22) Date of filing: 30.06.2025

(51) International Patent Classification (IPC):
*G01N 35/00* (2006.01)

(52) Cooperative Patent Classification (CPC):
G01N 35/00623; G16H 10/40; G16H 20/00;
G16H 50/20; G16H 50/70

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 28.06.2024 CN 202410868559
11.06.2025 CN 202510784615

(71) Applicant: Shenzhen Mindray Bio-Medical
Electronics Co., Ltd.
Shenzhen, Guangdong 518057 (CN)

(72) Inventors:
• ZHENG, Wenbo
Shenzhen, 518057 (CN)
• CHEN, Pengzhen
Shenzhen, 518057 (CN)
• YE, Bo
Shenzhen, 518057 (CN)
• QI, Huan
Shenzhen, 518057 (CN)

(74) Representative: KIPA AB
Drottninggatan 11
252 21 Helsingborg (SE)

(54) **METHOD, SAMPLE TESTING SYSTEM, DEVICE, AND MEDIUM FOR QUALITY CONTROL OF TESTING INSTRUMENT**

(57) Embodiments of the disclosure relate to a method for quality control of a testing instrument, a sample testing system, a device, and a computer-readable storage medium. The method includes: obtaining a quality control test result of a quality control material that simulates a biological sample, and obtaining a first monitoring indicator based on the quality control test result; determining whether the testing instrument is out of control based on the first monitoring indicator, outputting a first alarm prompt when the testing instrument is out of control, obtaining actual test results of a plurality of actual biological samples tested by the testing instrument for the target item, and inputting the actual test results into a patient-based real-time quality control (PBRTQC)-based quality control model to obtain second monitoring indicators; and determining whether the first alarm prompt is reliable based on the second monitoring indicators. This reduces an IQC false alarm probability.

**100**

FIG. 1

EP 4 671 769 A1

# Description

## TECHNICAL FIELD

The disclosure relates to the field of quality control of *in vitro* diagnostic instruments, and in particular to a method for quality control of a testing instrument, a sample testing system, a device for quality control of a testing instrument, and a computer-readable storage medium.

## BACKGROUND

During clinical diagnosis and treatment of diseases, medical laboratory quality directly affects the diagnosis and treatment of diseases by doctors. High-quality medical laboratory results rely on a complete quality control system for a medical laboratory. Quality control in a medical laboratory includes three aspects: pre-analytical quality control, analytical quality control, and post-analytical quality control. The analytical quality control is the most critical process in a quality management system. The analytical quality control includes internal quality control (IQC). A currently common practice of implementing IQC is as follows: a laboratory technician tests quality control materials at certain frequency, uses statistical theories to calculate and evaluate the reliability of test results, and then observes and eliminates out-of-control factors in the test. However, IQC monitoring performed by using quality control materials exhibits some typical drawbacks: 1) the process of IQC monitoring performed by using quality control materials is not continuous, but IQC monitoring is performed at individual time points to estimate whether the analytical process is out of control; 2) the quality control materials are patient samples, and therefore, may produce a matrix effect that affects IQC results; and 3) IQC performed by using quality control materials requires additional costs such as quality control materials, reagents, and human resources, and is thus costly.

In addition, the Analytical Quality Committee of the International Federation of Clinical Chemistry and Laboratory Medicine published a guideline in 2020 stating that: patient-based real-time quality control (PBRTQC) is a quality control method that uses test results of patient samples to monitor an analytical process of testing continuously in real time, and exhibits the advantages of cost-effectiveness, no matrix effect, real-time monitoring, etc. Despite such advantages, PBRTQC is still rarely used in clinical laboratories currently, and is not widely accepted by laboratory physicians for lack of unified standards for implementing PBRTQC.

## SUMMARY

An object of the disclosure is to provide a method for quality control of a testing instrument and a corresponding sample testing system, so as to maximally reduce false alarms of out-of-control events of the testing instrument, and in particular, to determine whether a quality control material is abnormal.

In order to achieve the object, a first aspect of the disclosure provides a method for quality control of a testing instrument, the method including:

obtaining a quality control test result of a quality control material that simulates a biological sample, and obtaining a first monitoring indicator based on the quality control test result, where the quality control test result is obtained by the testing instrument testing the quality control material for a target item at a quality control time point;

determining whether the testing instrument is out of control based on the first monitoring indicator; when the testing instrument is out of control, outputting a first alarm prompt indicating that the testing instrument is out of control, and obtaining actual test results of a plurality of actual biological samples tested by the testing instrument for the target item, and inputting the actual test results into a patient-based real-time quality control (PBRTQC)-based quality control model to obtain second monitoring indicators, where obtaining actual test results of a plurality of actual biological samples tested by the testing instrument for the target item comprises: 1) obtaining the actual test results of the plurality of actual biological samples tested by the testing instrument for the target item within a preset period of time before and/or after the quality control time point, or 2) obtaining actual test results of a preset number of biological samples tested by the testing instrument for the target item before and/or after the quality control time point; and

determining whether the first alarm prompt is reliable based on the second monitoring indicator.

In order to achieve the object, a second aspect of the disclosure provides a sample testing system, including: a testing instrument configured to test a test sample for a target item; and a controller communicatively connected to the testing instrument and configured to implement the method according to the first aspect of the disclosure.

In order to achieve the object, a third aspect of the disclosure provides a device for quality control of a testing instrument, the device including: a non-transitory computer-readable storage medium with computer-readable instructions stored thereon; one or more processors configured to execute the computer-readable instructions to implement the method according to the first aspect of the disclosure.

In order to achieve the object, a fourth aspect of the disclosure provides a computer-readable storage medium with a computer program stored thereon, the computer program, when executed by a processor, implementing the method according to the first aspect of the disclosure.

In the technical solutions provided in various aspects of the disclosure, quality control is performed on the testing instrument with reference to the IQC quality control result and the PBRTQC quality control result, thereby effectively reducing false alarms of out-of-control events of the testing instrument, especially indicating IQC false alarms.

## BRIEF DESCRIPTION OF THE DRAWINGS

FIG. 1 is a schematic flowchart of a method for quality control of a testing instrument according to an embodiment of the disclosure.

FIG. 2 is a schematic display interface for displaying monitoring indicators according to an embodiment of the disclosure.

FIG. 3 is another schematic display interface for displaying monitoring indicators according to an embodiment of the disclosure.

FIG. 4 is a schematic diagram of obtaining the number of patients until error detection.

FIG. 5 is a schematic graph of second monitoring indicators obtained according to an embodiment of the disclosure vs second monitoring indicators obtained according to the prior art.

FIG. 6 is a schematic flowchart of a method for constructing a quality control model for real-time quality control based on patient samples according to an embodiment of the disclosure.

FIG. 7 is a schematic flowchart of a method for optimizing a quality control model according to an embodiment of the disclosure.

FIG. 8 is a schematic flowchart of another method for optimizing a quality control model according to an embodiment of the disclosure.

FIG. 9 is a schematic flowchart of still another method for optimizing a quality control model according to an embodiment of the disclosure.

FIG. 10 is a schematic graph of second monitoring indicators obtained according to different embodiments of the disclosure.

FIG. 11 is a schematic diagram of grouping patient samples based on a clinical decision level of FT4 according to an embodiment of the disclosure.

FIG. 12 is a schematic block diagram of a sample testing system according to an embodiment of the disclosure.

FIG. 13 is a schematic diagram of another sample testing system according to an embodiment of the disclosure.

## DESCRIPTION OF THE PREFERRED EMBODIMENTS

The technical solutions of the embodiments of the disclosure will be described below clearly and comprehensively with reference to the accompanying drawings of the embodiments of the disclosure. Apparently, the embodiments described are merely some of, rather than all of, the embodiments of the disclosure. Based on the embodiments of the disclosure, all the other embodiments which would have been obtained by those of ordinary skill in the art without any creative efforts shall fall within the scope of protection of the disclosure.

As mentioned in the background, quality control based on quality control materials is characterized by the ability to immediately learn whether a testing instrument is out of control based on comparison between a single quality control test result and a reference value. However, because of high cost of the quality control materials, the number of tests of the quality control materials per day is limited, and is mostly 1 to 3 tests per day. Moreover, if the testing instrument is out of control during a test, the out-of-control event is not detectable until a next quality control test based on a quality control material, thereby posing a clinical risk. In addition, the quality control materials are subject to a shelf life, and a quality control material approaching the expiration date may result in a false report of an out-of-control event of the testing instrument.

Patient-based real-time quality control (PBRTQC) is a model or rule for real-time monitoring of laboratory test quality, which is built based on test results of patient samples by using a statistical model. The PBRTQC method is characterized by the ability to track the status of the testing instrument in real time with the progress of test of patient samples, and to monitor in real time whether the testing instrument is out of control. However, for the PBRTQC method, when the testing instrument is out of control, a specified number of samples need to be tested additionally before an out-of-control alarm can be raised.

In view of this, the disclosure proposes, based on the advantages of the two internal quality control methods, an internal quality control monitoring method that integrates real-time quality control and quality-control-material-based quality control.

In particular, an embodiment of the disclosure provides a technical solution to verify credibility of IQC quality control alarms based on PBRTQC quality control.

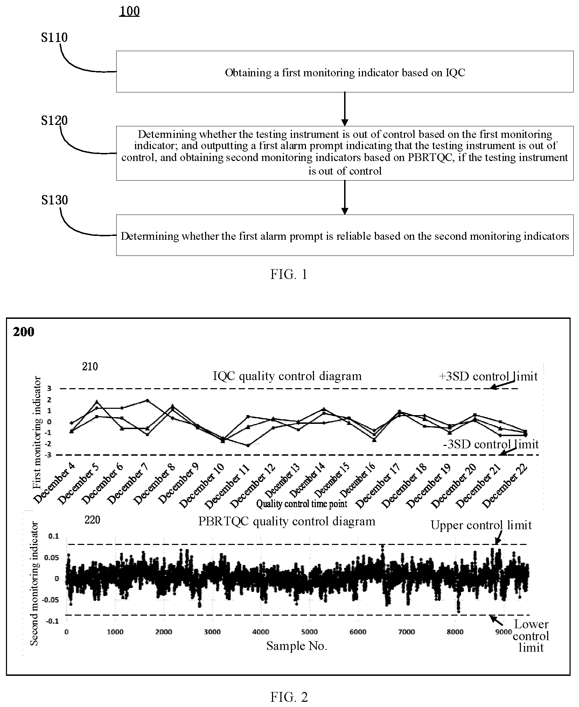

As shown in FIG. 1, an embodiment of the disclosure provides a method 100 for quality control of a testing instrument. The method 100 includes the following steps S110, S120, and S130.

At step S110, a first monitoring indicator based on a quality control material or based on IQC is obtained, that is, a quality control test result of a quality control material that simulates a biological sample is obtained, and a first monitoring indicator is obtained based on the quality control test result, where the quality control test result is obtained by the testing instrument testing the quality control material for a target item at a quality control time point.

In the embodiments of the disclosure, the quality control material is a product or substance used in a laboratory for quality control, which is similar to a patient sample. In other words, the quality control material is a product that is specially manufactured for quality control of instruments, and is different from a biological sample obtained from a subject such as a human body.

In some embodiments, the quality control time point may include one or more fixed time points in each week, or the quality control time point may include one or more fixed time points in each day. For example, the quality control time point includes a power-on time point and/or a power-off time point of the testing instrument every day.

In other embodiments, the quality control time point may include a time point after the testing instrument has tested a preset number of actual biological samples.

At step S120, it is determined whether the testing instrument is out of control based on the first monitoring indicator. If the testing instrument is out of control, a first alarm prompt indicating that the testing instrument is out of control is output, and second monitoring indicators based on PBRTQC are obtained, that is, actual test results of a plurality of actual biological samples tested by the testing instrument for the target item are obtained, and inputted into a PBRTQC-based quality control model, so as to obtain second monitoring indicators, where the plurality of actual biological samples include biological samples tested by the testing instrument within a preset period of time before and/or after the quality control time point, or the plurality of actual biological samples include a preset number of biological samples tested by the testing instrument before and/or after the quality control time point.

Here, particularly, the actual test results are obtained by the testing instrument through continuous testing over time of the plurality of actual biological samples for the target item.

In the embodiments of the disclosure, an actual biological sample refers to a sample, such as a blood sample, obtained directly from a subject such as a human body.

At step S130, it is determined whether the first alarm prompt is reliable based on the second monitoring indicator.

By combining the first monitoring indicator based on the quality control material with the second monitoring indicators based on PBRTQC to perform quality control on the testing instrument, it is possible to reduce false alarms of the testing instrument, especially IQC false alarms, while reducing clinical risk.

In the embodiments of the disclosure, the testing instrument being in control or under control means that the testing instrument is in a normal state and has no fault; and the testing instrument being out of control means that the testing instrument is in an abnormal state and may have a fault.

In some embodiments, the actual test results may be obtained by the testing instrument testing the plurality of actual biological samples within 24 hours before or after the quality control time point. This allows for more accurate quality control.

Further, the plurality of actual biological samples may include a preset number of biological samples tested by the testing instrument within a preset period of time before and/or after the quality control time point. For example, the plurality of actual biological samples may include a preset number, such as 100 to 200, of biological samples tested by the testing instrument within 24 hours before and/or after the quality control time point.

In some implementations, the actual test results are obtained by the testing instrument testing the plurality of actual biological samples within 24 hours, preferably 18 hours, before the quality control time point. In other words, when it is determined that the testing instrument is out of control based on the first monitoring indicator, test results of historical biological samples are retrieved to obtain the second monitoring indicators, and it is determined, based on the second monitoring indicators, whether the testing instrument is really out of control or whether the first alarm prompt is reliable. If it is determined that the testing instrument is out of control based on the second monitoring indicators, it is determined that the first alarm prompt is reliable; otherwise, it is determined that the first alarm prompt is unreliable.

In another implementation, the actual test results are obtained by the testing instrument testing the plurality of actual biological samples within 2 hours after the quality control time point. In other words, when it is determined that the testing instrument is out of control based on the first monitoring indicator, a plurality of actual biological samples are still tested for the target item within a preset period of time, and second monitoring indicators are obtained based on test results. For example, if it is determined that the testing instrument is out of control based on the second monitoring indicators, it is determined that the first alarm prompt is reliable, and the actual biological samples are re-tested after the abnormality of the testing instrument is eliminated; otherwise, it is determined that the first alarm prompt is unreliable.

In some embodiments, at step S130, determining whether the first alarm prompt is reliable based on the second monitoring indicators, may include:

determining, when it is determined based on the second monitoring indicators that the testing instrument is out of control, that the first alarm prompt is reliable, and preferably outputting a second alarm prompt indicating that the testing instrument is out of control.

In some embodiments, at step S130, determining whether the first alarm prompt is reliable based on the second monitoring indicators, may include:

determining, when it is determined based on the second monitoring indicators that the testing instrument is in control, that the first alarm prompt is unreliable, and outputting a third alarm prompt indicating that the first alarm prompt is unreliable.

In other words, the out-of-control alarm, that is, the first alarm prompt, based on the first monitoring indicator, is suspicious and may be a false alarm in this case.

In some embodiments, at step S130, when it is determined that the first alarm prompt is unreliable, prompt information indicating that a possible reason for unreliability of the first alarm prompt lies in the quality control material rather than the testing instrument may be further output. In other words, the IQC alarm may be raised due to an abnormality of the quality control material. In this case, prompt information indicating abnormality of the quality control material may be output, so that the user is prompted to check whether the quality control material is abnormal, for example, to check shelf-life expiry or deterioration of the quality control material.

In some embodiments, when it is determined that the first alarm prompt is unreliable, prompt information indicating that the second monitoring indicators should be used as a quality control reference may be further output.

In some embodiments, the method 100 may further include:

outputting, when it is determined that the first alarm prompt is unreliable, a prompt to perform quality control again by using the quality control material or by using another quality control material, preferably by using another quality control material in a different batch from the quality control material, or, controlling dispatching of the quality control material or another quality control material to the testing instrument for test, so as to obtain another quality control test result of the quality control material or the another quality control material for the target item.

In other words, when the first alarm prompt is unreliable, another quality control may be performed by using the quality control material or the another quality control material, so as to check whether the testing instrument is really out of control.

In some embodiments, the method 100 is performed by a processor. The processor is a processor of the testing instrument, or, the processor is independent of the testing instrument and is communicatively connected to the testing instrument.

In some embodiments, a dispatching device may be controlled to retrieve the quality control material or the another quality control material from a storage device and to transfer the quality control material or the another quality control material to the testing instrument for testing, so as to obtain the another quality control test result.

In some embodiments, after outputting the prompt to perform quality control again by using the quality control material or the another quality control material, the method 100 may further include: controlling, in response to an input operation of a user with respect to the prompt, the testing instrument to test the quality control material or the another quality control material for the target item, so as to obtain the another quality control test result.

For example, the user manually confirms to perform IQC and manually sends the quality control material or the another quality control material to the testing instrument for testing. After the instruction to perform IQC is received, the testing instrument is controlled to test the quality control material or the another quality control material.

For another example, the user manually confirms to perform IQC. After the instruction to perform IQC is received, the quality control material or the another quality control material is automatically dispatched to the testing instrument for testing. For example, the dispatching device is controlled to retrieve the quality control material or the another quality control material from the storage device and to transfer the quality control material or the another quality control material to the testing instrument for testing, so as to obtain the another quality control test result.

Next, some implementations of output displaying of the first monitoring indicator and the second monitoring indicators are described below.

In some embodiments, the method 100 may further include: displaying, for example, controlling to display, the first monitoring indicator and the second monitoring indicators on the same display interface.

By displaying the IQC-based first monitoring indicator and the PBRTQC-based second monitoring indicators on the same display interface, the disclosure enables visual observation of change trend of the IQC-based first monitoring indicator and the PBRTQC-based second monitoring indicators, so as to determine whether the testing instrument is out of control based on both indicators.

In some embodiments, the method 100 further includes: labeling, on the display interface, the actual biological sample, if it is determined that the testing instrument is out of control based on the first monitoring indicator, and on the second monitoring indicators associated with the actual biological sample.

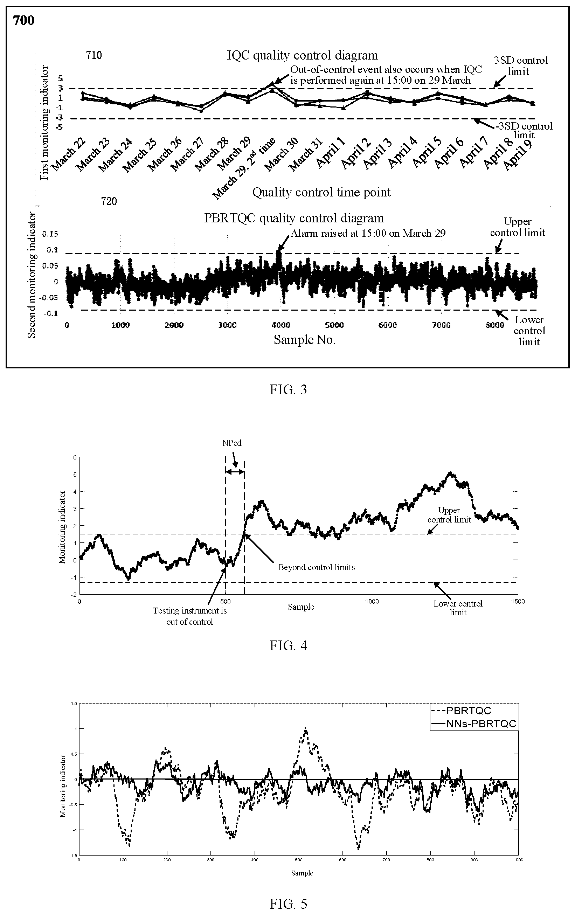

In a specific example, as shown in FIG. 2 and FIG. 3, displaying, for example, controlling to display, the first monitoring indicator and the second monitoring indicators on the same display interface, may include:

displaying, for example, controlling to display, the first monitoring indicator in a first coordinate system 210 on the same display interface 200, where an ordinate of the first coordinate system represents the first monitoring indicator and an abscissa of the first coordinate system represents a test time point of the quality control material; and
displaying, for example, controlling to display, the second monitoring indicators in a second coordinate system 220 on the same display interface 200, where an ordinate of the second coordinate system represents the second monitoring indicators, and an abscissa of the second coordinate system represents a test time point of each of the plurality of actual biological samples or a serial number of each of the plurality of actual biological samples or test order of the plurality of actual biological samples.

FIG. 2 shows a display interface when the target item is an albumin (ALB) parameter of liver functions, where the display interface 200 shows an IQC-based quality control chart and a PBRTQC-based monitoring chart from December 4, 2022 to December 12, 2022. The upper part of FIG. 2 is the IQC-based quality control chart, in which an alarm will be raised when the IQC-based first monitoring indicator exceeds three standard deviations (3SD). The lower part of FIG. 2 is the PBRTQC-based monitoring chart, in which an alarm will be raised when at least one of the second monitoring indicators exceeds a control limit. In FIG. 2, no out-of-control alarm is generated based on the IQC-based first monitoring indicator and based on the PBRTQC-based second monitoring indicators.

FIG. 3 shows another display interface when the target item is an ALB parameter of liver functions, in which the display interface 700 shows an IQC-based quality control chart and a PBRTQC-based monitoring chart from March 22, 2023 to April 9, 2023. The upper part of FIG. 3 is the IQC-based quality control chart, in which an alarm will be raised when the IQC-based first monitoring indicator exceeds three standard deviations (3SD). The lower part of FIG. 3 is the PBRTQC-based monitoring chart, in which an alarm will be raised when at least one of the second monitoring indicators exceeds a control limit. In FIG. 3, an alarm is generated based on the PBRTQC-based second monitoring indicators at 15:00 p.m. on March 29, IQC is performed after the alarm is generated, and it is found that the testing instrument is out of control based on the IQC. After the out-of-control of the instrument is solved, no out-of-control alarm is generated based on IQC and based on PBRTQC on March 30.

In other implementations, displaying the first monitoring indicator and the second monitoring indicators on a same display interface, may include: displaying the first monitoring indicator and the second monitoring indicators in a same coordinate system on the same display interface, where an ordinate of the same second coordinate system represents the first monitoring indicator and the second monitoring indicators, and an abscissa of the same coordinate system represents a test time point of the quality control material and a test time point of each of the plurality of actual biological samples.

In some embodiments, the method 100 further includes: smoothing and/or filtering the second monitoring indicators before displaying the second monitoring indicators.

How to obtain the PBRTQC-based second monitoring indicators is described below with reference to some embodiments.

In some implementations, the PBRTQC-based quality control model includes a statistical process control (SPC) algorithm-based computational model, for example is the SPC algorithm-based computational model. Accordingly, the second monitoring indicators are obtained at step S120 by: inputting the actual test results into the SPC algorithm-based computational model, and using output results of the computational model as the second monitoring indicators. For example, at step S 120, data processing, such as truncation processing and normal transformation (for example, Box-Cov transformation), is performed on the actual test results, and then the actual test results subjected to the data processing are used as input of the SPC algorithm so that calculation results of the SPC algorithm are obtained as the second monitoring indicators. Here, upper and lower control limits of the second monitoring indicators may be determined by setting a false alarm rate and using test results obtained when the testing instrument is in control as the modeling data of the SPC algorithm-based computational model. During the PBRTQC-based real-time monitoring, if the testing instrument is out of control, at least one of the SPC algorithm-based second monitoring indicators will exceed the control limit, and thus generating an alarm indicating that the testing instrument is out of control.

In some embodiments, the statistical process control SPC algorithm may include one of the following algorithms: moving average, moving median, exponentially weighted moving average, moving standard deviation, moving quantile and Moving Number of Positive Patients.

In some embodiments, parameters of the truncation processing and parameters of the computational model may be optimized by:

obtaining actual test results of a plurality of historical patient samples for the target item, where the actual test results are obtained by the testing instrument in a controlled state testing the historical patient samples;

setting a false alarm rate;

setting a plurality of truncation ratios for the truncation processing and a plurality of SPC parameter values of the computational model;

determining, in the following manner, upper and lower control limits at the false alarm rate, and an average number of patients until error detection, for each parameter value combination of each truncation ratio and each SPC parameter value:

calculating second monitoring indicators for said parameter value combination in the computational model with said parameter value combination by using the actual test results of the plurality of historical patient samples, and obtaining the upper and lower control limits based on the second monitoring indicators for said parameter value combination and based on the false alarm rate;

calculating monitoring indicators for said parameter value combination in presence of an instrument out-of-control event in the computational model with said parameter value combination by using a simulated data set containing a plurality of simulated instrument out-of-control events or a real data set containing a plurality of real instrument out-of-control events, and comparing the calculated monitoring indicators with the upper and lower control limits, so as to calculate an average number of samples required from the beginning of each simulated instrument out-of-control event or real instrument out-of-control event to a time point of actually detecting said simulated instrument out-of-control event or real instrument out-of-control event, that is, to calculate an average number of patients until error detection,

where the simulated data set is obtained by adding errors to the actual test results of the plurality of historical patient samples repeatedly at different time points, and the real data set is obtained from the actual test results of the plurality of historical patient samples and patient information thereof or obtained by obtaining actual test results of a plurality of additional historical patient samples for the target item and patient information thereof; and

selecting a parameter value combination with a minimum average number of patients until error detection and upper and lower control limits corresponding to the selected parameter value combination, so as to build the optimized truncation processing and the optimized computational model.

In an example in which the SPC algorithm is a moving average method, the parameter of the moving average-based computational model is sliding window. Parameters to be optimized include the sliding window and the truncation ratio. The false alarm rate is set to 0.1%, the parameter value of the sliding window is set to 5, 10, 15, 20, 50, and 100, and the parameter value of the truncation ratio for the truncation processing is set to $\pm$ 0%, $\pm$ 1%, $\pm$ 2%, and $\pm$ 5%. Second monitoring indicators at different parameter value combinations (in this example, there are a total of 24 parameter value combinations) of the sliding window and the truncation ratio are calculated by using the actual test results of the plurality of historical patient samples. Subsequently, upper and lower control limits are calculated based on the second monitoring indicators and the false alarm rate (for example, if the second monitoring indicators are calculated by using the actual test results of N patient samples for the target item, then the number of false alarms is N*0.1%, and both the number of false alarms below the upper control limit and the number of false alarms above the lower control limit are N*0.1%/2. Accordingly, the upper and lower control limits are identified, where the number of monitoring indicators beyond the upper control limit and the number of monitoring indicators beyond the lower control limit are N*0.1%/2). Subsequently, errors are randomly added to the actual test results of the plurality of historical patient samples, and a number of patients until error detection for each added error is calculated, and then the calculated values are averaged out to obtain an average number of patients until error detection, thereby determining the parameter value combination satisfying the specified false alarm rate and a minimum average number of patients until error detection.

In other implementations, the PBRTQC-based quality control model includes a machine learning model. In other words, the second monitoring indicators may be obtained based on the machine learning model.

In particular, for patients with diseases, the test results thereof fluctuate significantly, and PBRTQC in the related art requires a large number of consecutive samples for identifying an error and a system out-of-control state, which cannot meet clinical IQC requirements. In particular, the inventors of the disclosure have found that, for conventional indicators such as white blood cell count, red blood cell count, hemoglobin content and thyroid stimulating hormone, factors such as disease, age and gender of a patient lead to wide distribution and high fluctuation of the conventional indicators, thereby resulting in a large average number of patients until error detection of a testing system based on the PBRTQC method in the related art.

Therefore, an embodiment of the disclosure further provides a technical solution to performing real-time quality control based on patient samples and a machine learning model, especially a neural network model, so as to reduce the impact of factors such as disease, age and gender of a patient on PBRTQC quality control performance (that is, to improve quality control stability) while reducing the number of patients until error detection compared to existing PBRTQC quality control (that is, increasing sensitivity).

In some embodiments, at step S120, inputting the actual test results into a PBRTQC-based quality control model to obtain second monitoring indicators includes:

obtaining patient information of actual patients corresponding to the plurality of actual biological samples, where the patient information includes at least one of disease, age, gender and an ordering department of the target item; quantifying the patient information of actual patients corresponding to the plurality of actual biological samples, and inputting the quantified patient information into the machine learning model, and using output results of the machine learning model as predicted test results for the target item, where the machine learning model is obtained by training with actual test results of biological samples of a plurality of historical patients for the target item and patient information of the plurality of historical patients; and

obtaining the second monitoring indicators based on the actual test results and the predicted test results of the plurality of actual biological samples.

The performance of the PBRTQC-based and machine learning model-based quality control method of the disclosure and the performance of the PBRTQC-based quality control method in the prior art are verified by using the number of patients until error detection NPed at the same false alarm rate. A smaller number of patients until error detection indicates a higher sensitivity of error detection. The false alarm rate is a ratio of the number of false alarms (the number of alarms generated by the monitoring indicators beyond a preset control limit) generated based on monitoring indicators calculated from data obtained by the testing instrument in a controlled state to the total number of samples. The number of patients until error detection is the number of samples required from occurrence of an out-of-control event of the testing instrument to a time point of at least one monitoring indicator exceeding the preset control limit, as shown in FIG. 4.

The quality control method is verified using an example in which the target items are white blood cell count WBC, red blood cell count RBC, hemoglobin content HGB, and thyroid stimulating hormone TSH. The PBRTQC-based quality control method of the disclosure and the PBRTQC-based quality control method of the prior art are simulated by way of adding errors repeatedly to a WBC verification data set, an RBC verification data set, an HGB verification data set, and a TSH verification data set that are obtained through the testing instrument in a controlled state, so as to obtain the average number of patients until error detection ANPed shown in Table 1. As shown in Table 1, the PBRTQC-based quality control method disclosed in the disclosure significantly improves the quality control sensitivity in contrast to the prior art.

Table 1 ANPed for different target items

| Target item | PBRTQC of the prior art | PBRTQC of the disclosure |
|---|---|---|
| WBC | 229 | 89 |
| RBC | 170 | 91 |
| HGB | 181 | 81 |
| TSH | 410 | 116 |

Furthermore, at the same alarm rate and taking that the target item is white blood cell count (WBC) as an example, monitoring indicators (solid line) in an embodiment of the disclosure and monitoring indicators (dashed line) of existing PBRTQC are calculated respectively based on the WBC data set obtained through the testing instrument in a controlled state, as shown in FIG. 5. As can be seen from FIG. 5, the monitoring indicators calculated according to this embodiment of the disclosure are more stable than the monitoring indicators calculated according to the existing PBRTQC. In addition, at the same alarm rate, the control limit obtained according to this embodiment of the disclosure is smaller than the control limit obtained according to the existing PBRTQC.

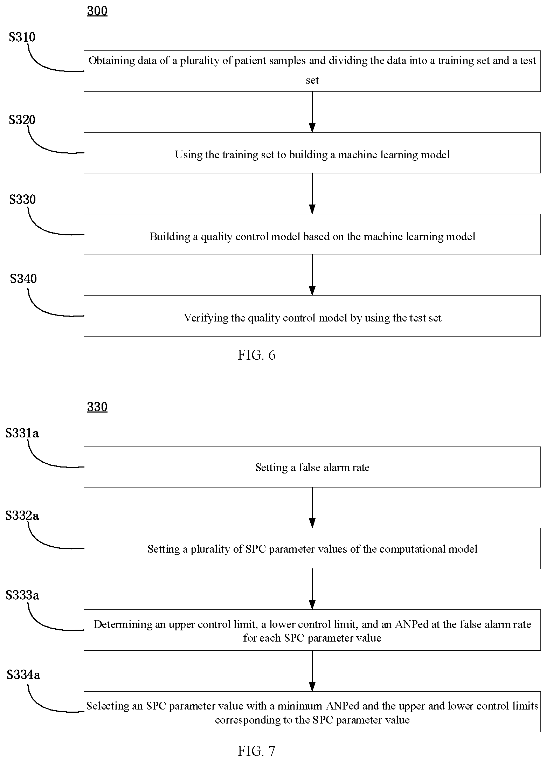

In some embodiments, the quality control model for real-time quality control based on patient samples may be built by using a method 300 shown in FIG. 6. The method 300 includes the following steps.

S310: Obtaining data of a plurality of (historical or known) patient samples and dividing the data into a training set and a test set (for example, dividing into a training set and a test set in chronological order). The data includes actual test results of the plurality of patient samples for the target item and patient information of the plurality of patient samples. The actual test results are obtained by the testing instrument testing the patient samples. The patient information includes at least one of disease, age, gender and a department to which the sample belongs. The data of the training set is obtained when the testing instrument is in a controlled state. Here, for example, actual test results of a plurality of patient samples, especially all patient samples, which are tested by the testing instrument for the target item in chronological order over a period of time, as well as the patient information thereof, are obtained.

At step S310, the sample ratio of the training set to the test set may be set to 6 : 4 or 7 : 3, or the like.

S320: Using the training set to build a machine learning model by quantifying the patient information of the patient samples in the training set, and building a machine learning model between the quantified patient information and the actual test results in the training set, where the machine learning model satisfies the following formula:

$$X_t = f(x_{1t}, x_{2t}, \cdots, x_{nt}) + \varepsilon_t$$

where $X_t$ is an actual test result in the training set, $f(x_{1t}, x_{2t}, \cdots, x_{nt})$ is the machine learning model, $x_{1t}, x_{2t}, \cdots, x_{nt}$ represent the quantified patient information, and $\varepsilon_t$ represents a residual estimated by using the machine learning model $X_t$. In other words, the machine learning model $f$ is trained by performing regression learning on the historical test results of the testing instrument in a controlled state and the patient information. The $\varepsilon_t$ at this time approximately assumes a random distribution with a mean value of 0.

S330: Building, based on the machine learning model, the quality control model that is used for performing real-time quality control based on patient samples.

S340: Verifying the quality control model by using the test set, so as to verify the performance of the quality control model.

The machine learning model $f$ is an artificial neural network nonlinear function, and reasonably estimates deviations caused by factors such as disease, age and gender. After such factors affecting parameter fluctuations are eliminated, monitoring indicators obtained by using the quality control model built based on the machine learning model are stable, thereby improving system error detection performance in contrast to the PBRTQC in the prior art.

When the testing instrument is in a normally controlled state, the residual $\varepsilon_t$ fluctuates randomly, and the sum-of-squares error is minimal. In some embodiments, a loss function $min\Sigma\|\varepsilon_t\|^2$ may be created based on this principle. A machine learning model $f(x_{1t}, x_{2t}, \cdots, x_{nt})$ with a minimized loss function is calculated by using the actual test results of the training samples (that is, the plurality of patient samples) and the patient information as input.

In some embodiments, an average of the actual test results of n patient samples for the target item may be used as a variable of the machine learning model, n being a value such as a daily test flux of the testing instrument, and the n patient samples being tested prior to a specified time point among the plurality of (historical or known) patient samples. This reduces daytime impact caused by factors such as calibration of the testing instrument and daytime impact caused by reagents.

The machine learning model is trained by using the actual test results of a plurality of known patient samples (that is, historical patient samples) for the target item and the patient information. Here, the machine learning model may further characterize relationships between the patient information, the mean value of the actual test results of the plurality of known patient samples, and the test results of the target item. In other words, if the patient information is input into the machine learning model, a predictive test result for the target item predicted by the machine learning model will be obtained.

In some embodiment, the machine learning model may be a neural network model. In other embodiments, the machine learning model may be a machine learning model based on a support vector machine (SVM) and/or linear discriminant analysis (LDA).

In some embodiments, the target item may be a blood routine examination item, for example, cell count (such as a white blood cell count, a platelet count, a red blood cell count) and cell classification (such as white blood cell classification), and hemoglobin content. Accordingly, the testing instrument is a blood cell analyzer.

In other embodiments, the target item may be a biochemical test item, for example, thyroid function (such as thyroid stimulating hormone), liver function, kidney function, blood lipids, blood glucose, creatinine (Cr), urea (UA), thyroid stimulating hormone (TSH), and free thyroxine (FT4). Accordingly, the testing instrument is a biochemical analyzer.

In some embodiment, the patient information may include multiple of disease, age, gender and a department to which the sample belongs. Preferably, the patient information may include disease, age, gender and a department to which the sample belongs.

In the embodiments of the disclosure, the department to which the sample belongs means an ordering department of the target item, that is, a hospital department that requests to use the testing instrument to test an actual biological sample for the target item.

In some embodiment, the patient information may be obtained from a laboratory (clinical laboratory department) information system of a hospital in which the testing instrument is located.

In some embodiments, obtaining the second monitoring indicators based on the actual test results and the predicted test results of the plurality of actual biological samples may include:

calculating differences between the actual test results and the predicted test results of the plurality of actual biological samples; and
inputting the differences into a statistical process control (SPC) algorithm-based computational model, and using output results of the computational model as the second monitoring indicators.

The statistical process control SPC algorithm may include, for example, at least one of moving average, moving median, exponentially weighted moving average, moving standard deviation, moving quantile and Moving Number of Positive Patients.

In some embodiments, as shown in FIG. 7, based on the embodiment shown in FIG. 6, parameters of the computational model may be optimized at step S330 by performing the following steps:

step S331a: setting a false alarm rate FAR;

step S332a: setting a plurality of SPC parameter values of the computational model;

step S333a: determining upper and lower control limits at the false alarm rate, and an average number of patients until error detection for each SPC parameter value in the following manner:

calculating monitoring indicators for said SPC parameter value in the quality control model with said SPC parameter value by using the actual test results and the patient information in the training set, and obtaining the upper and lower control limits based on the monitoring indicators at said SPC parameter value and based on the false alarm rate,

calculating monitoring indicators for said parameter value in presence of an instrument out-of-control event in the quality control model with said SPC parameter value by using a simulated data set containing a plurality of simulated instrument out-of-control events or a real data set containing a plurality of real instrument out-of-control events, and comparing the calculated monitoring indicators with the upper and lower control limits, so as to calculate an average number of samples required from the beginning of each simulated instrument out-of-control event or real instrument out-of-control event to a time point of actually detecting said simulated instrument out-of-control event or real instrument out-of-control event by the quality control model, that is, to calculate an average number of patients until error detection ANPed, where the simulated data set is obtained by repeatedly adding errors to the actual test results in the training set at different time points, and the real data set is obtained from the data of the plurality of patient samples or obtained by obtaining actual test results of a plurality of additional patient samples for the target item and the patient information thereof; and

step S334a: selecting an SPC parameter value with a minimum average number of patients until error detection and the upper and lower control limits corresponding to the selected SPC parameter value, so as to build an optimized quality control model.

Here, the false alarm rate directly determines the upper and lower control limits. The false alarm rate is usually set manually by a user in view of the usage situation. For example, the false alarm rate is set to 0.1%. Once the false alarm rate is determined, the upper and lower control limits can also be determined.

Here, it should be understood that values of other parameters of the quality control model such as a truncation ratio are fixed during optimization of the parameters of the computational model. For example, the fixed values are selected empirically.

Preferably, the average number of patients until error detection ANPed is calculated by using the simulated data set containing the plurality of simulated instrument out-of-control events, as follows: calculating monitoring indicators for said SPC parameter value in the quality control model with said SPC parameter value by using the actual test results and the patient information in the training set, and obtaining upper and lower control limits based on the monitoring indicators for said parameter value and based on the false alarm rate; and adding, at different time points repeatedly, errors to the actual test results of the patient samples in the training set for the target item; inputting the actual test results of the patient samples for the target item in the training set together with the corresponding patient information into the quality control model with said SPC parameter value after adding the errors, so as to obtain monitoring indicators after the addition of the errors; and comparing the monitoring indicators with the upper and lower control limits, so as to calculate an average number of samples required from the beginning of each added error to a time point of actually detecting said error by the quality control model, this is, to calculate an average number of patients until error detection ANPed.

In an example in which the SPC algorithm is a moving average method, the parameter of the moving average-based computational model is sliding window. The false alarm rate is set to 0.1%, and the parameter value of the sliding window is set to 5, 10, 15, 20, 50, and 100. Monitoring indicators for different sliding windows are calculated by using the training set. Subsequently, upper and lower control limits are calculated based on the monitoring indicators and the false alarm rate (for example, if the monitoring indicators are calculated by using the actual test results of N patient samples for the target item as well as the patient information, then the number of false alarms is N*0.1%, and both the number of false alarms below the upper control limit and the number of false alarms above the lower control limit are N*0.1 %/2. Accordingly, the upper control limit and the lower control limit are identified. Both the number of monitoring indicators beyond the upper control limit and the number of monitoring indicators beyond the lower control limit are N*0.1%/2). Subsequently, errors are added randomly at a plurality of positions in the training set. The number of patients until error detection for each added error is calculated, and then the calculated values are averaged out to obtain an average number of patients until error detection, thereby determining the parameter value satisfying the specified false alarm rate and a minimum average number of patients until error detection.

In some embodiments, steps S331a, S332a, S333a, and S334a may be repeated, and then a minimum average number of patients until error detection ANPedmin is selected from the average numbers of patients until error detection at a plurality of false alarm rates FAR (for example, 0.001%, 0.01%, 0.1%, 1%, 3%, and 5%) to obtain a parameter value corresponding to the selected minimum average number of patients until error detection and upper and lower control limits corresponding to said parameter value, so as to build an optimized quality control model. In other embodiments, a false alarm rate FAR and a minimum average number of patients until error detection ANPedmin at this false alarm rate FAR may also be weighted and then summed, expressed as: sum = FAR * a + ANPedmin * b, where a is the weight of the FAR and b is the weight of the minimum average number of patients until error detection ANPedmin. The sum of each false alarm rate FAR and a minimum average number of patients until error detection ANPedmin at this false alarm rate FAR is calculated, and then the minimum average number of patients until error detection corresponding to a minimum value of the sum and a corresponding parameter value are selected.

In other embodiments, if the test set contains data of an out-of-control event of the testing instrument, then upper and lower control limits and an average number of patients until error detection at the false alarm rate may be determined for each SPC parameter value by using the test set as the real data set in the following manner: calculating monitoring indicators for said the SPC parameter value in the quality control model with said SPC parameter value by using the actual test results of the patient samples for the target item in the training set as well as the patient information, and obtaining the upper and lower control limits based on the monitoring indicators for said SPC parameter value and based on the false alarm rate; inputting the actual test results of the patient samples for the target item in the test set together with the corresponding patient information into the quality control model with said SPC parameter value, so as to obtain monitoring indicators corresponding to the test set; and comparing the monitoring indicators with the upper and lower control limits, so as to calculate an average number of samples required from the beginning of each instrument out-of-control event to a time point of actually detecting said out-of-control event by the quality control model, this is, to calculate an average number of patients until error detection ANPed.

In still other embodiments, another test set may be obtained. The other test set includes actual test results of a plurality of patient samples for a target item as well as patient information. The actual test results are obtained by the testing instrument testing the patient samples. The patient information includes at least one of disease, age, gender and a department to which the sample belongs. The other test set includes data of out-of-control events of the testing instrument. Here, upper and lower control limits and an average number of patients until error detection at the false alarm rate may be determined for each SPC parameter value by using the other test set in the following manner: calculating monitoring indicators for sad the SPC parameter value in the quality control model with said SPC parameter value by using the actual test results of the patient samples for the target item in the training set as well as the patient information, and obtaining the upper and lower control limits based on the monitoring indicators for said SPC parameter value and based on the false alarm rate; inputting the actual test results of the patient samples for the target item in the other test set together with the corresponding patient information into the quality control model with said SPC parameter value, so as to obtain monitoring indicators corresponding to the other test set; and comparing the monitoring indicators with the upper and lower control limits, so as to calculate the average number of samples required from the beginning of each instrument out-of-control event to a time point of actually detecting said out-of-control event by the quality control model, this is, to calculate an average number of patients until error detection ANPed.

In some embodiments, truncation processing, and optionally normal transformation, may be performed on the actual test results of the patient samples for the target item in the training set before building the machine learning model.

In other embodiments, obtaining the second monitoring indicators based on the actual test results and the predicted test results may also include: obtaining the second monitoring indicators based on a ratio of the actual test results to the predicted test results.

In some embodiments, obtaining the second monitoring indicators based on the actual test results and the predicted test results may include:

performing data processing on the actual test results, where the data processing includes truncation processing and/or normalization processing; and
obtaining the second monitoring indicators based on the actual test results that are subjected to the data processing and the predicted test results.

However, in the embodiments employing the machine learning model, the actual test results may be not normalized. This simplifies data processing.

In some embodiment, a fixed truncation ratio may be set for the truncation processing.

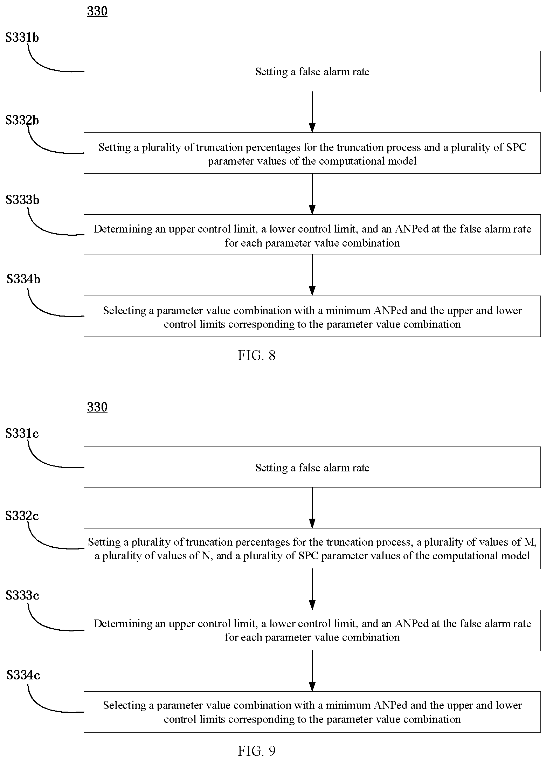

In some embodiments, as shown in FIG. 8, at step S330, parameters of the truncation processing and parameter of the computational model may be optimized by:

step S331b: setting a false alarm rate FAR;

step S332b: setting a plurality of truncation ratios for the truncation processing and a plurality of SPC parameter values of the computational model;

step S333b: determining, in the following manner, upper and lower control limits and an average number of patients until error detection at the false alarm rate for each parameter value combination of each truncation ratio and each SPC parameter value:

calculating monitoring indicators at said parameter value combination in the quality control model with said parameter value combination by using the actual test results and the patient information in the training set, and obtaining the upper and lower control limits based on the monitoring indicators for said parameter value combination and based on the false alarm rate, and

calculating monitoring indicators for said parameter value combination in presence of an instrument out-of-control event in the quality control model with said parameter value combination by using a simulated data set containing a plurality of simulated instrument out-of-control events or a real data set containing a plurality of real instrument out-of-control events, and comparing the calculated monitoring indicators with the upper and lower control limits, so as to calculate an average number of samples required from the beginning of each simulated instrument out-of-control event or real instrument out-of-control event to a time point of actually detecting said simulated instrument out-of-control event or real instrument out-of-control event by the quality control model, that is, to calculate an average number of patients until error detection,

where the simulated data set is obtained by adding errors to the actual test results in the training set repeatedly at different time points, and the real data set is obtained from the data of the plurality of patient samples or obtained by obtaining actual test results of a plurality of additional patient samples for the target item and the patient information thereof; and

step S334b: selecting a parameter value combination with a minimum average number of patients until error detection and the upper and lower control limits corresponding to the selected parameter value combination, so as to build an optimized quality control model.

Here, it should be understood that values of other parameters of the quality control model are fixed during optimization of the parameters of the truncation processing and the parameters of the computational model. For example, the fixed values are selected empirically.

Preferably, the average number of patients until error detection ANPed is calculated by using the simulated data set containing the plurality of simulated instrument out-of-control events, as follows: calculating monitoring indicators for said parameter value in the quality control model with said parameter value combination by using the actual test results of the patient samples for the target item in the training set as well as the patient information, and obtaining the upper and lower control limits based on the monitoring indicators for said parameter value and based on the false alarm rate; adding, at different time points repeatedly, errors to the actual test results of the patient samples in the training set for the target item; inputting the actual test results of the patient samples for the target item in the training set together with the corresponding patient information into the quality control model with said parameter value combination after adding the errors, so as to obtain monitoring indicators after the addition of the errors; and comparing the monitoring indicators with the upper and lower control limits, so as to calculate an average number of samples required from the beginning of each added error to a time point of actually detecting said error by the quality control model, this is, to calculate an average number of patients until error detection ANPed.

Likewise, in some embodiments, steps S331b, S332b, S333b and S334b may be repeated, and then an minimum average number of patients until error detection is selected from the average numbers of patients until error detection at a plurality of false alarm rates (for example, 0.001%, 0.01%, 0.1%, 1%, 3% and 5%) to obtain a parameter value combination corresponding to the selected minimum average number of patients until error detection and upper and lower control limits corresponding to said parameter value combination, so as to build an optimized quality control model. In other embodiments, a false alarm rate FAR and a minimum average number of patients until error detection ANPedmin at this false alarm rate FAR may also be weighted and then summed, expressed as: sum = FAR * a + ANPedmin * b, where a is the weight of the FAR and b is the weight of the minimum average number of patients until error detection ANPedmin. The sum of each false alarm rate FAR and a minimum average number of patients until error detection ANPedmin at this false alarm rate FAR is calculated, and then a minimum average number of patients until error detection corresponding to a minimum value of the sum and a corresponding parameter value combination are selected.

In an example in which the process control SPC algorithm is a moving average method, the parameter of the moving average-based computational model is sliding window. Parameters to be optimized of the quality control model include sliding window and truncation ratio. The false alarm rate is set to 0.1%, the parameter value of the sliding window is set to 5, 10, 15, 20, 50, and 100, and the parameter value of the truncation ratio for the truncation processing is set to $\pm$ 0%, $\pm$ 1%, $\pm$ 2%, and $\pm$ 5%. Monitoring indicators at different parameter value combinations (in this example, there are a total of 24 parameter value combinations) of the sliding window and the truncation ratio are calculated by using the training set. Subsequently, upper and lower control limits are calculated based on the monitoring indicators and the false alarm rate (for example, if the monitoring indicators are calculated by using the actual test results of N patient samples for the target item as well as patient information, then the number of false alarms is N*0.1%, and both the number of false alarms below the upper control limit and the number of false alarms above the lower control limit are N*0.1%/2. Accordingly, the upper and lower control limits are identified. Both the number of monitoring indicators beyond the upper control limit and the number of monitoring indicators beyond the lower control limit are N*0.1%/2). Subsequently, errors are added randomly at a plurality of positions in the training set. The number of patients until error detection for each added error is calculated, and then the calculated values are averaged out to obtain an average number of patients until error detection, thereby determining a parameter value combination satisfying the specified false alarm rate and a minimum average number of patients until error detection.

In some embodiments, quantifying the patient information of the actual patients corresponding to the plurality of actual biological samples may include: for each of the actual biological samples,
quantifying at least one type of the patient information of the actual patient corresponding to said actual biological sample, especially at least one of a disease, a gender and an ordering department of the target item, into a matrix.

In an example, quantifying at least one type of the patient information of the actual patients corresponding to the plurality of actual biological samples into a matrix may include: for each of the actual biological samples,
quantifying each type of patient information in the at least one type of patient information of said actual biological sample into a one-dimensional matrix containing a plurality of elements, where said type of patient information of said actual biological sample belongs to at least one of a plurality of categories, each element of the one-dimensional matrix represents one of the plurality of categories, and different elements correspond to different categories.

For example, when the patient information includes disease information, if diseases are classified into 33 disease categories, the disease information may be quantified as a one-dimensional matrix containing 33 elements, where each element represents one disease.

For another example, when the patient information includes gender information, gender is divided into 2 categories, i.e., male and female, and therefore the gender information may be quantified as a one-dimensional matrix containing 2 elements, in which one element represents male, and the other element represents female.

Still for another example, when the patient information includes information about the department to which the sample belongs, if departments to which the samples belong are classified into 32 department categories, the information about the department to which the sample belongs may be quantified as a one-dimensional matrix containing 32 elements. Each element represents one department.

Preferably, quantifying each type of patient information in the at least one type of patient information of said actual biological sample into a one-dimensional matrix containing a plurality of elements may include:
quantifying each type of patient information in the at least one type of patient information of said actual biological sample into a one-dimensional matrix containing a plurality of elements, so that the value of an element representing the category of said type of patient information of said actual biological sample is set to 1, and the values of remaining elements are set to 0. This simplifies the building of the machine learning model.

For example, when the patient information includes disease information, if diseases are classified into 33 disease categories, the disease information may be quantified as a one-dimensional matrix $[X1, X2, ..., X33]$ containing 33 elements, in which each element X represents one disease. For example, X1 represents hyperthyroidism, X2 represents hypertension, and so on. If the disease information of an actual biological sample is hyperthyroidism, then the disease information of the patient sample is quantified as $[1, 0, 0, 0, ..., 0]$, in which the value of the element X1 is 1, and the values of the remaining elements are 0. If the disease information of an actual biological sample includes hyperthyroidism and hypertension, the disease information of the patient sample is quantified as $[1, 1, 0, 0, ..., 0]$, in which the values of elements X1 and X2 are 1, and the values of the remaining elements are 0.

For another example, when the patient information includes gender information, gender is divided into 2 categories, i.e., male and female, and therefore the gender information may be quantified as a one-dimensional matrix $[Y1, Y2]$ containing 2 elements, in which the element Y1 represents male, and the element Y2 represents female. If the gender information of a patient sample is male, then the gender information of the patient sample is quantified as $[1, 0]$, in which the value of the element Y1 is 1, and the value of the element Y2 is 0.

Still for another example, when the patient information includes information about the department to which the sample belongs, if departments to which the samples belong are classified into 32 department categories, the information about the department to which the sample belongs may be quantified as a one-dimensional matrix $[Z1, Z2, ..., Z32]$ containing 32 elements. Each element represents one department. For example, Z1 represents endocrinology department, and Z2 represents nephrology department. If the information about the department to which a patient sample belongs is endocrinology department, the information of the department to which the patient sample belongs is quantified as [1,0, 0, 0, ..., 0], in which the value of the element Z1 is 1, and the values of the remaining elements are 0.

In other embodiments, quantifying the patient information of the actual patients corresponding to the plurality of actual biological samples may include: for each of the actual biological samples, quantifying at least one type of the patient information of the actual patient corresponding to said actual biological sample, especially age, into a fixed value, preferably into a fixed integer value.

In an example, age may be quantified as a fixed value of 0 to 150. To be specific, if a patient is 30 years old, then the age is quantified as 30. In other examples, age may be quantified as a percentage. For example, if the patient is 30 years old, then the age is quantified as 30/100.

In an example, gender may be quantified as a first positive integer or a second positive integer, where the first positive integer represents male, and the second positive integer represents female. For example, the first positive integer is 1, and the second positive integer is 2, but the disclosure is not limited to the example.

Alternatively or additionally, quantifying the patient information of the actual patients corresponding to the plurality of actual biological samples may include: for each of the actual biological samples, quantifying at least one type of the patient information of the actual patient corresponding to said actual biological sample by using a table lookup method. This simplifies the building of the machine learning model.

In an example, different positive integers may be set for various corresponding diseases, and the correspondence between each disease and the corresponding positive integer is pre-stored in the form of a lookup table. Therefore, during quantification of the disease of a patient, the lookup table is searched for the positive integer corresponding to the disease of the patient. Table 2 shows an example of a lookup table used for quantifying diseases.

Table 2 Lookup table for quantifying diseases

| Clinical diagnosis | Value |
|---|---|
| Hypertension | 1 |
| Diabetes | 2 |
| Medical examination | 3 |
| Uremia; Peritoneal dialysis therapy | 4 |
| Prostate cancer | 5 |
| … | … |

In an example, different positive integers may be set for various corresponding departments, and the correspondence between each department and the corresponding positive integer is pre-stored in the form of a lookup table. Therefore, during quantification of the department to which the patient sample belongs, the lookup table is searched for the positive integer corresponding to the department to which the patient sample belongs. Table 3 shows an example of a lookup table used for quantifying departments.

Table 3 Lookup table for quantifying departments

| Department | Value |
|---|---|
| Radiation therapy outpatient department | 1 |
| Chemotherapy outpatient department | 2 |
| Infectious diseases outpatient department | 3 |
| Hematology outpatient department | 4 |
| Gynecology outpatient department | 5 |
| … | … |

Here, it should be understood that the quantification of the patient information of the historical patient samples, also referred to as known patient samples, during the building of the quality control model is performed in the same way as the quantification of the patient information corresponding to the plurality of actual biological samples described above, and will not be described here again.

In some embodiment, at step S140, it may be determined that the testing instrument is out of control and an alarm prompt may be output, when one of the second monitoring indicators exceeds a preset control limit.

14

In other embodiments, at step S140, it may be determined that the testing instrument is out of control and an alarm prompt may be output, when M consecutive second monitoring indicators (obtained in chronological order) exceed a preset control limit, where M is a natural number greater than 1. This can reduce the false alarm rate in contrast to a practice in which an alarm is raised when one second monitoring indicator exceeds the preset control limit.

In still other embodiments, at step S140, second monitoring indicators of a first number M of consecutive actual biological samples may be obtained in chronological order, and it is determined whether the testing instrument is in control based on the second monitoring indicators of the first number M of consecutive actual biological samples. It is determined that the testing instrument is out of control, and an alarm prompt indicating that the testing instrument is out of control, is output, only when the second monitoring indicators of at least a second number N of actual biological samples among the first number M of consecutive current patients (in chronological order) exceed a preset control limit, where both the first number M and the second number N are natural numbers greater than 1, and the first number M is greater than the second number N. In this way, false alarms can be distinguished from true alarms significantly. This can reduce the false alarm rate in contrast to a practice in which an alarm is raised when one monitoring indicator exceeds the preset control limit while minimizing the number of patients until error detection.

In other words, determining whether the testing instrument is out of control or whether the first alarm prompt is reliable based on the second monitoring indicators, includes:

obtaining second monitoring indicators of a first number M of consecutive actual biological samples in chronological order, and determining whether the testing instrument is in control based on the second monitoring indicators of the first number M of consecutive actual biological samples;

when the second monitoring indicators of at least the second number N of actual biological samples among the first number M of consecutive actual biological samples exceed a control limit, determining that the testing instrument is out of control, where the first number M is greater than the second number N, and the second number N is greater than 1.

In some embodiments, as shown in FIG. 9, M and N may also be parameters to be optimized of the quality control model. To be specific, the parameters of the computational model, M, N, and optionally the parameters of the truncation processing may be optimized at step S330 in the following manner:

S331c: setting a false alarm rate;

S332c: setting a plurality of SPC parameter values of the computational model, a plurality of parameter values of M, a plurality of parameter values of N, and optionally a plurality of truncation ratios for the truncation processing;

S333c: determining, in the following manner, upper and lower control limits and an average number of patients until error detection at the false alarm rate for each parameter value combination of each SPC parameter value, each parameter value of M, each parameter value of N, and optionally each truncation ratio:

calculating monitoring indicators for said parameter value combination in the quality control model with said parameter value combination by using the actual test results and the patient information in the training set, and obtaining upper and lower control limits based on the monitoring indicators for said parameter value combination and based on the false alarm rate, and

calculating monitoring indicators for said parameter value combination in presence of an instrument out-of-control event in the quality control model with said parameter value combination by using a simulated data set containing a plurality of simulated instrument out-of-control events or a real data set containing a plurality of real instrument out-of-control events, and comparing the calculated monitoring indicators with the upper and lower control limits, so as to calculate an average number of samples required from the beginning of each simulated instrument out-of-control event or real instrument out-of-control event to a time point of actually detecting said simulated instrument out-of-control event or real instrument out-of-control event by the quality control model, that is, to calculate an average number of patients until error detection,

where the simulated data set is obtained by adding errors to the actual test results in the training set repeatedly at different time points, and the real data set is obtained from the data of the plurality of patient samples or obtained by obtaining actual test results of a plurality of additional patient samples for the target item and the patient information thereof; and

S334c: selecting a parameter value combination with a minimum average number of patients until error detection and the upper and lower control limits corresponding to the selected parameter value combination, so as to build an optimized quality control model.

Here, it should be understood that values of other parameters of the quality control model are fixed during optimization of the parameters of the computational model, M, N, and optionally the parameters of the truncation processing. For example, the fixed values are selected empirically. For example, the truncation ratio for the truncation processing may be fixed during optimization of the parameters of the computational model, M, and N.

Likewise, in some embodiments, steps S331c, S332c, S333c and S334c may be repeated, and then an minimum average number of patients until error detection is selected from the average numbers of patients until error detection at a plurality of false alarm rates (for example, 0.001%, 0.01%, 0.1%, 1%, 3% and 5%) to obtain a parameter value combination corresponding to the minimum average number of patients until error detection and upper and lower control limits corresponding to the parameter value combination, so as to build an optimized quality control model. In other embodiments, a false alarm rate FAR and a minimum average number of patients until error detection ANPedmin at this false alarm rate FAR may also be weighted and then summed, expressed as: sum = FAR * a + ANPedmin * b, where a is the weight of the FAR and b is the weight of the minimum average number of patients until error detection ANPedmin. The sum of each false alarm rate FAR and a minimum average number of patients until error detection ANPedmin at this false alarm rate FAR is calculated, and then a minimum average number of patients until error detection corresponding to a minimum value of the sum and a corresponding parameter value combination are selected.

Preferably, the average number of patients until error detection ANPed is calculated by using the simulated data set containing the plurality of simulated instrument out-of-control events, as follows: calculating monitoring indicators for said parameter value in the quality control model with said parameter value combination by using the actual test results and the patient information in the training set, and obtaining upper and lower control limits based on the monitoring indicators for said parameter value and based on the false alarm rate; and adding, at different time points repeatedly, errors to the actual test results of the patient samples in the training set for the target item; inputting the actual test results of the patient samples for the target item in the training set together with the corresponding patient information into the quality control model with said parameter value combination after adding the errors, so as to obtain monitoring indicators after the addition of the errors; and comparing the monitoring indicators with the upper and lower control limits, so as to calculate an average number of samples required from the beginning of each added error to a time point of actually detecting said error by the quality control model, this is, to calculate an average number of patients until error detection ANPed.

In an example in which the SPC algorithm is a moving average method, the parameter of the moving average-based computational model is sliding window. Parameters to be optimized of the quality control model include sliding window, truncation ratio, M, and N. The false alarm rate is set to 0.1%, the parameter value of the sliding window is set to 10, 20, 50, 70, and 100, the parameter value of the truncation ratio for the truncation processing is set to $\pm$ 0%, $\pm$ 1%, $\pm$ 2%, and $\pm$ 5%, and M and N are set to the parameter values specified in Table 4. Monitoring indicators at different parameter value combinations of the sliding window, the truncation ratio, M, and N are calculated by using the training set, and then upper and lower control limits are calculated based on the monitoring indicators and the false alarm rate. Subsequently, errors are added randomly at a plurality of positions in the training set. A number of patients until error detection for each added error is calculated, and then the calculated values are averaged out to obtain an average number of patients until error detection. The false alarm rate is reset to a plurality of different values such as 0.001%, 0.01%, 1%, 3%, and 5%, and the above process is repeated to determine a parameter value combination that satisfies the minimum average number of patients until error detection.

Table 4 Parameter values of alarm parameters

| N | M |
|---|---|
| 1 | 3 |
| 3 | 5 |
| 7 | 10 |
| 14 | 20 |
| … | … |

In other embodiments not shown, M and N may also be parameters to be optimized of the quality control model. The parameters of the computational model, M, N, and optionally the parameters of the truncation are optimized at step S330 in the following manner:

setting a plurality of control limit positions of the quality control model, a plurality of SPC parameter values of the computational model, a plurality of alarm parameter value combinations M and N, and optionally a plurality of truncation ratios for the truncation processing;
determining, in the following manner, a false alarm rate and an average number of patients until error detection for each parameter value combination of each control limit position, each SPC parameter value, each alarm parameter value combination, and optionally each truncation ratio:

calculating monitoring indicators for said parameter value combination in the quality control model with said parameter value combination by using the actual test results and the patient information in the training set, and calculating a false alarm rate of the model based on the upper and lower control limits in said parameter value combination and the monitoring indicators for said parameter value combination, and

calculating monitoring indicators for said parameter value combination in presence of an instrument out-of-control event in the quality control model with said parameter value combination by using a simulated data set containing a plurality of simulated instrument out-of-control events or a real data set containing a plurality of real instrument out-of-control events, and comparing the calculated monitoring indicators with the control limits in said parameter value combination, so as to calculate an average number of samples required from the beginning of each simulated instrument out-of-control event or real instrument out-of-control event to a time point of actually detecting said simulated instrument out-of-control event or real instrument out-of-control event by the quality control model, that is, to calculate an average number of patients until error detection,

where the simulated data set is obtained by adding errors to the actual test results in the training set repeatedly at different time points, and the real data set is obtained from the data of the plurality of patient samples or obtained by obtaining actual test results of a plurality of additional patient samples for the target item and the patient information thereof; and

determining an optimal parameter value combination based on the false alarm rate of the model and the average number of patients until error detection for each parameter value combination, so as to build an optimized quality control model.

In some embodiments, a parameter value combination that contributes to a minimum average number of patients until error detection among the parameter value combinations with a false alarm rate lower than a preset false alarm rate of the model may be selected as a final optimized parameter value combination for the quality control model.

In other embodiments, the false alarm rate of the model and the average number of patients until error detection for each parameter value combination may be weighted, and then added up to obtain a sum, and then a parameter value combination corresponding to a minimum value of the sum is selected.

In an example in which the SPC algorithm is a moving average method, the parameter of the moving average-based computational model is sliding window. Parameters to be optimized of the quality control model include sliding window, truncation ratio, and M&N alarm parameter combination. The upper and lower control limit positions (located at the upper and lower S/2 percentiles of the monitoring indicators respectively, where S is 0.001%, 0.01%, 0.1%, 1%, 3%, 5%) are set. The parameter value of the sliding window is set to 10, 20, 50, 70, and 100. The parameter value of the truncation ratio for the truncation processing is set to $\pm$ 0%, $\pm$ 1%, $\pm$ 2%, and $\pm$ 5%. The alarm parameter combination is set to the parameter values specified in Table 4. Monitoring indicators for different parameter value combinations of the upper and lower control limits, the sliding window, the truncation ratio, and the alarm parameter combination are calculated by using the training set, and then a false alarm rate of the model is calculated based on the monitoring indicators and the upper and lower control limits. Subsequently, errors are added randomly at a plurality of positions in the training set. A number of patients until error detection for each added error is calculated, and then the calculated values are averaged out to obtain an average number of patients until error detection. A parameter value combination that contributes to a minimum average number of patients until error detection among the parameter value combinations with a false alarm rate lower than a preset false alarm rate of the model is selected as a final optimized parameter value combination for the quality control model.

In still other embodiments not shown, M and N may also be parameters to be optimized of the quality control model. The control limits of the quality control model, the parameters of the computational model, M, N, and optionally the parameters of the truncation are optimized in the following manner:

setting a false alarm rate of the quality control model, a plurality of control limit positions, a plurality of SPC parameter values of the computational model, a plurality of alarm parameter value combinations M and N, and optionally a plurality of truncation ratios for the truncation processing;

determining, in the following manner and a false alarm rate for each parameter value combination of each control limit position, each SPC parameter value, each alarm parameter value combination, and optionally each truncation ratio:

calculating monitoring indicators for said parameter value combination in the quality control model with said parameter value combination by using the actual test results and the patient information in the training set, and calculating a false alarm rate of the model based on the control limit positions in said parameter value combination and the monitoring indicators for said parameter value combination; and

selecting each model parameter value combinations with a false alarm rate lower than the preset false alarm rate of the model as a candidate parameter value combination;

determining an average number of patients until error detection for each candidate parameter value combination in the following manner: calculating monitoring indicator for said candidate parameter value combination in presence of an instrument out-of-control event in the quality control model with said candidate parameter value combination by using a simulated data set containing a plurality of simulated instrument out-of-control events or a real data set containing a plurality of real instrument out-of-control events, and comparing the calculated monitoring indicators with the control limits in said candidate parameter value combination, so as to calculate an average number of samples required from the beginning of each simulated instrument out-of-control event or real instrument out-of-control event to a time point of actually detecting said simulated instrument out-of-control event or real instrument out-of-control event by the quality control model, that is, to calculate an average number of patients until error detection, where the simulated data set is obtained by repeatedly adding errors to the actual test results in the training set at different time points, and the real data set is obtained from the data of the plurality of patient samples or obtained by obtaining actual test results of a plurality of additional patient samples for the target item and the patient information thereof; and

using a candidate parameter value combination with a minimum average number of patients until error detection to build an optimized quality control model.

Here, it should be understood that values of other parameters of the quality control model are fixed during optimization of the control limits of the quality control model, the parameters of the computational model, M, N, and optionally the parameters of the truncation. For example, the fixed values are selected empirically. For example, the truncation ratio for the truncation processing may be fixed during optimization of the control limits of the quality control model, the parameters of the computational model, M, and N.

In some embodiments, at step S340, the quality control model may be verified by inputting the data of the test set into the quality control model built at step S330, determining whether the corresponding monitoring indicators fall within the upper and lower control limits, and if at least one of the monitoring indicators beyond the upper or lower control limit, it indicates that a non-random deviation occurs in the system, and that the testing instrument is out of control, and therefore, an out-of-control alarm is generated. If the monitoring indicators are not beyond the upper and lower control limits, it indicates that the system is normal and the testing instrument is in control. If data about instrument out-of-control status exists in the test set, the data may be used directly to verify the validity of the algorithm. In the case where no data about out-of-control status of the testing instrument exists in the test set, the testing performance of the algorithm model may be verified by adding allowable errors in the test set from a specified time point to simulate data about out-of-control status of the testing instrument. The simulation is performed repeatedly (adding errors to the test set at different time points), and all simulated numbers of patients until error detection can be averaged out to obtain an average number of patients until error detection. The performance of the quality control model is verified by using the average number of patients until error detection.

In some embodiments, fitting of the PBRTQC-based machine learning model is difficult and complicated. In view of this situation, an embodiment of the disclosure further provides a real-time quality monitoring solution based on patient sample grouping, which enables a PBRTQC-based machine learning model that can be easily fitted.

In some implementations, at step S 120, inputting the actual test results into a PBRTQC-based quality control model to obtain second monitoring indicators may include: for each of the plurality of actual biological samples,

selecting a group matching said actual biological sample from a plurality of groups, and preferably selecting a group matching said actual biological sample from the plurality of groups based on a preset criterion, where the plurality of groups are divided based on the preset criterion; and

inputting the actual test result of said actual biological sample into a quality control model corresponding to the selected group to obtain the second monitoring indicator.

Therefore, the actual biological samples are grouped based on the preset criterion to determine the group to which each actual biological sample belongs. Subsequently, based on the group to which said actual biological sample belongs, a corresponding matching PBRTQC-based machine learning model is identified. Therefore, based on said machine learning model, a second monitoring indicator for determining whether the testing instrument is controlled is obtained. In this way, the method for real-time quality control based on patient samples disclosed herein can be implemented in a simple way.

The method is verified using an example in which the target items are creatinine (Cr), urea (UA), thyroid stimulating hormone (TSH), and free thyroxine (FT4). The sample grouping-based PBRTQC quality control method and the quality control method based on the embodiment shown in FIG. 6 are simulated by way of adding errors repeatedly to a Cr verification data set, an UA verification data set, an TSH verification data set, and an FT4 verification data set that are obtained through the testing instrument in a controlled state, so as to obtain the average number of patients until error detection ANPed shown in Table 5. As shown in Table 5, the sample grouping-based PBRTQC quality control method significantly improves the quality control sensitivity compared to the quality control method based on the embodiment shown in FIG. 6.

Table 5 ANPed of different target items

| Target item | PBRTQC of FIG. 6 | sample grouping-based PBRTQC |
|---|---|---|
| Cr | 87 | 45 |
| UA | 228 | 95 |
| TSH | 410 | 120 |
| FT4 | 29 | 17 |

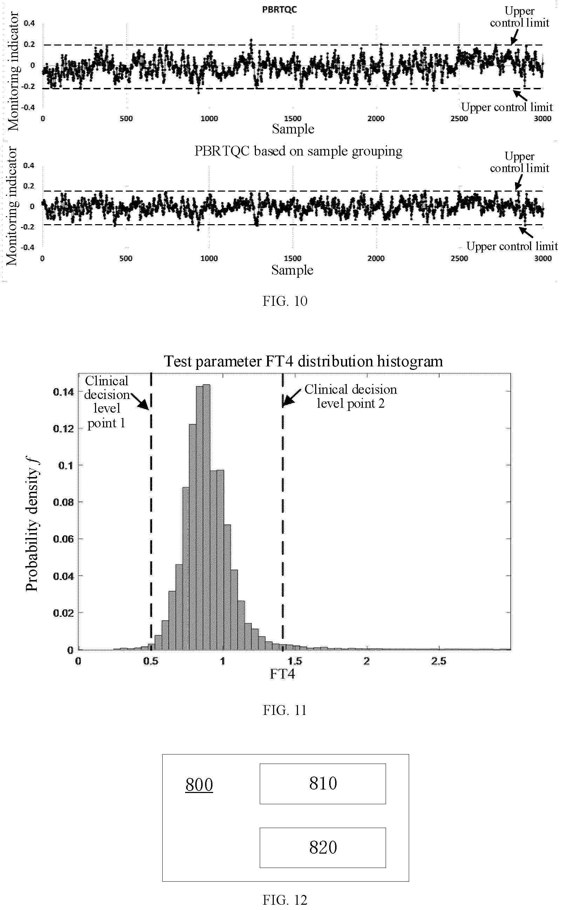

Furthermore, at the same alarm rate and taking that the target item is FT4 as an example, monitoring indicators based on the sample grouping and monitoring indicators based on the embodiment shown in FIG. 6 are calculated separately based on the FT4 data set obtained through the testing instrument in a controlled state, as shown in FIG. 10 (the upper part of FIG. 10 shows the monitoring indicators based on the embodiment shown in FIG. 6, and the lower part of FIG. 10 shows the monitoring indicators based on the sample grouping). As can be seen from FIG. 10, the monitoring indicators based on the sample grouping are more stable than the monitoring indicators based on the embodiment shown in FIG. 6. In addition, at the same alarm rate, the control limit obtained based on sample grouping is smaller than the control limit obtained based on the embodiment shown in FIG. 6.

In some embodiments, the plurality of groups and the preset criterion are predetermined correlatively and pre-stored in a memory of a computer system, for example, in the form of a lookup table.

In some embodiment, the actual biological samples may be grouped based on the actual test results of the actual biological samples. For this purpose, the preset criterion includes a plurality of threshold ranges of a test result for the target item.

In an example, the plurality of threshold ranges correspond one-to-one to the plurality of groups. Accordingly, selecting a group matching said actual biological sample from a plurality of groups based on a preset criterion includes: selecting, from the plurality of threshold ranges, a threshold range within which the actual test result of said actual biological sample falls, and using a group corresponding to the selected threshold range as the group matching said actual biological sample.

In other words, each threshold range corresponds to a group. For example, the preset criterion includes a first threshold range S1, a second threshold range S2, and a third threshold range S3 different from each another. An actual biological sample with an actual test result for the target item falling within the first threshold range belongs to a first group; an actual biological sample with an actual test result for the target item falling within the second threshold range belongs to a second group; and an actual biological sample with an actual test result for the target item falling within the third threshold range belongs to a third group.

In some embodiments, the preset criterion includes a reference range or a clinical decision level of a test result of the target item. In other words, the plurality of threshold ranges are obtained based on the reference range or the clinical decision level.

It should be understood that the reference range of a test result for the target item is an interval obtained based on data statistics of test results of a healthy population or a specific population, and is used for reflecting the distribution of test results of most normal individuals in a specific physiological state, and is generally a distribution range of test results of 95% of normal population. In other words, the reference range is generally obtained through statistical analysis on the test results of a healthy population or a specific population, and is mainly based on the physiological characteristics of the population and the distribution law of the test results. The reference range is an interval that extends for a specific breadth, and is used for covering test results of most normal individuals and reflecting the physiological variability of normal population. The reference range is primarily intended to provide a reference standard for clinicians to determine whether a test result is abnormal, and to further consider the possibility of a disease.

It should be understood that the clinical decision level is a test result threshold relevant to a clinical decision-making and having specific clinical significance. When a test result reaches or exceeds the clinical decision level, it may indicate that some specific clinical intervention is required, for example, starting treatment, adjusting a treatment regimen, and initiating close follow-up. In other words, the clinical decision level is generally based on extensive clinical studies and established practical experience, and allows for a combination of factors such as the natural course of the disease, the effects of the treatment method, and cost-effectiveness. The clinical decision level is typically one or more specific numerical values, which has definite clinical significance and directive value for decision-making. For example, the clinical decision level of serum creatinine may be used for determining whether renal replacement therapy is required. The clinical decision level is primarily used for guiding clinicians in making decisions and is a direct basis for disease diagnosis, treatment, and prognosis evaluation. For example, the clinical decision level of blood sugar can help clinicians determine whether a patient needs an insulin therapy.

It should be understood that the reference range is a basis of the clinical decision level, and the clinical decision level is usually determined with reference to the reference range. The reference range provides clinicians with an initial standard for judgment, and helps gain a general understanding of test results. The clinical decision level complements and refines the reference range. The reference range itself cannot directly guide clinical decisions, whereas the clinical decision level, based on the reference range and in consideration of the clinical characteristics of the disease and treatment needs, further clarifies the relationship between test results and clinical interventions, and offers clinicians more targeted and operational guidance. In actual clinical diagnosis and treatment, a clinician usually compares a test result with the reference range, initially determines whether the result is abnormal, and then determines, based on the clinical decision level, whether corresponding clinical measures need to be taken. The reference range and the clinical decision level complement each other and together provide a basis for clinical decision-making.

In some implementations, the plurality of threshold ranges are obtained based on the reference range of a test result for the target item. For example, the first threshold range S1 is a range less than a reference range, the second threshold range S2 is the reference range, and the third threshold range S3 is a second range greater than the reference range. Using free thyroxine (FT4) in serum as an example, the reference range of a test result for the target item is 0.7 to 1.8 ng/dL, and therefore, the first threshold range S1 is less than 0.7 ng/dL, the second threshold range S2 is 0.7 to 1.8 ng/dL, and the third threshold range S3 is greater than 1.8 ng/dL. In this case, a patient sample with a test result of FT4 being less than 0.7 ng/dl belongs to a first group, a patient sample with a test result of FT4 falling between 0.7 ng/dl and 1.8 ng/dl belongs to a second group, and a patient sample with a test result of FT4 being greater than 1.8 ng/dl belongs to a third group.

In other implementations, the plurality of threshold ranges are obtained based on the clinical decision level of a test result for the target item. For example, FIG. 11 is a schematic diagram of patient sample grouping based on the clinical decision level of free thyroxine (FT4) in serum. Assuming that a clinical decision level point 1 of FT4 is 0.5 ng/dl and a clinical decision level point 2 of FT4 is 1.4 ng/dl, the first threshold range S1 is less than 0.5 ng/dL, the second threshold range S2 is 0.5 to 1.4 ng/dL, and the third threshold range S3 is greater than 1.4 ng/dL. In this case, a patient sample with a test result of FT4 being less than 0.5 ng/dl belongs to a first group, a patient sample with a test result of FT4 falling between 0.5 ng/dl and 1.4 ng/dl belongs to a second group, and a patient sample with a test result of FT4 being greater than 1.4 ng/dl belongs to a third group.

In other embodiments, the actual biological samples may be grouped based on patient information of the actual biological samples. In other words, the preset criterion includes patient information, where the patient information includes at least one of disease, age, gender, an ordering department of the target item and type of medical visit.

In the embodiments of the disclosure, the department to which the sample belongs means an ordering department of the target item, that is, a hospital department that requests to use the testing instrument to test an actual biological sample for the target item.

In embodiments of the disclosure, the type of medical visit indicates whether a patient is an inpatient or a non-inpatient, or, whether a patient is an inpatient, an outpatient, or an emergency patient.

In an example, the actual biological samples may be grouped based on disease of patients to which the actual biological samples belong. For example, the preset criterion includes a plurality of disease types, and each disease type corresponds to one group. For example, the preset criterion may include an internal medicine disease type and a surgical system disease type. Patient samples from patients with the internal medicine disease type belong to a first group, and patient samples from patients with the surgical system disease type belong to a second group.

In another example, the actual biological samples may be grouped based on age of patients to which the actual biological samples belong. For example, the preset criterion includes a plurality of age ranges, such as less than 17 years old, 18 to 45 years old, 46 to 69 years old, and over 70 years old, and each age range corresponds to one group. For example, patient samples from patients with an age less than 17 years old belongs to a first group, patient samples from patients with an age 18 to 45 years belong to a second group, patient samples from patients with an age of 46 to 69 years belong to a third group, and patient samples from patients with an age over 70 years belong to a fourth group.

In still another example, the actual biological samples may be grouped based on gender of patients to which the actual biological sample belong. For example, the preset criterion includes male and female. Male corresponds to a first group, and female corresponds to a second group.

In still other embodiments, the actual biological samples may be grouped based on the actual test results and the patient information of the actual biological samples. In other words, the preset criterion includes a plurality of threshold ranges of a test results for the target item and includes the patient information, where the patient information includes at least one of disease, age, gender, an ordering department of the target item and type of medical visit.

Here, a further balance between model complexity and model accuracy can be achieved by grouping current patient samples based on both the actual test results and the patient information of the current patient samples.

Here, it should be understood that the patient information used for grouping in the preset criterion is the same as the patient information used for selecting a matching group for the current patient. For example, in the case where the samples are grouped based on the plurality of preset thresholds and the patient disease, selecting a group matching said actual biological sample from the plurality of groups based on a preset criterion includes: selecting, based on the actual test result of the current patient sample and the disease of the current patient, a group that matches the current patient sample from the plurality of groups. For another example, in the case where the samples are grouped based on the plurality of preset thresholds and the ordering department of the patient, selecting a group matching said actual biological sample from the plurality of groups based on a preset criterion includes: selecting, based on the actual test result of the current patient sample and the ordering department of the current patient, a group that matches the current patient sample from the plurality of groups. For still another example, in the case where the samples are grouped based on the plurality of preset thresholds, the patient disease and the ordering department of the patient, selecting a group matching said actual biological sample from the plurality of groups based on a preset criterion includes: selecting, based on the actual test result of the current patient sample, the disease of the current patient, and the ordering department of the current patient, a group that matches the current patient sample from the plurality of groups.

In some embodiments, the preset criterion includes only one or two of disease, age, gender, an ordering department of the target item and type of medical visit.

In some implementations, the patient information may include only the disease or the ordering department of the target item. Accordingly, selecting a group matching said actual biological sample from a plurality of groups based on a preset criterion includes: selecting a group matching the current patient sample from the plurality of groups based only on the actual test result of the current patient sample and the disease of the current patient, or based only on the actual test result of the current patient sample and the ordering department of the target item of the current patient.

Here, the samples are grouped based on the disease or the ordering department of the target item additionally, so that the discrimination between the groups is greater, thereby enabling faster detection of errors.

In an example, the patient information may include only the disease of patient. In other words, the current patient samples may be grouped based only on the actual test results of the current patient samples and may be grouped based on the diseases of patients to which the current patient samples belong. For example, the disease of patient in the preset criterion includes a plurality of disease types, and each disease type corresponds to one group. For example, the preset criterion may include an internal medicine disease type and a surgical system disease type. The internal medicine disease type and the surgical system disease type each correspond to three threshold ranges, and therefore, six groups may be obtained based on such preset criterion. Patient samples from patients with the internal medicine disease type belong to a first group, and patient samples from patients with the surgical system disease type belong to a second group.

In another example, the patient information may include only the ordering department of the target item. In other words, the current patient samples may be grouped based only on the actual test results of the current patient samples and may be grouped based on the ordering departments of the target item of the current patient samples. For example, the ordering department of the target item in the preset criterion includes a plurality of ordering departments, and each group corresponds to at least one ordering department.

In still other embodiments, the patient information may include only the disease of patient and the ordering department of the target item. Accordingly, selecting a group matching said actual biological sample from a plurality of groups based on criteria preset criterion includes: selecting a group matching the current patient sample from the plurality of groups based only on the actual test result of the current patient sample, the disease of the current patient, and the ordering department of the target item of the current patient.

Here, the samples are grouped based on the disease of patient and the ordering department of the target item additionally, so that the discrimination between the groups is even greater, thereby enabling even faster detection of errors.

In some embodiments, the PBRTQC quality control model based on sample grouping may be built by:

obtaining data of a plurality of patient samples and dividing the data into a training set and a test set (for example, dividing into a training set and a test set in chronological order); here, the data includes actual test results of the plurality of patient samples for the target item, the actual test results are obtained by the testing instrument testing the patient samples, and the data in the training set is obtained when the testing instrument is in a controlled state. Here, for example, actual test results of a plurality of patient samples, especially all patient samples, which are tested by the testing instrument for the target item in chronological order over a period of time are obtained;

dividing the patient samples in the training set into a plurality of groups, and for each of the plurality of groups, building a machine learning model corresponding to said group based on the data of the patient samples belonging to said group;

building, based on the machine learning models corresponding to the plurality of groups, a quality control model that is used for performing real-time quality control based on patient samples; and

verifying the quality control model by using the test set.

Here, the patient samples in the training set are divided into the plurality of groups based on a preset criterion.

In some embodiments, the data of the plurality of patient samples further includes patient information of the plurality of patient samples. The patient information includes at least one of a disease, an age, a gender and an ordering department of the target item, and preferably the patient information includes a disease, an age, a gender and an ordering department to which the target item belongs. Building a machine learning model corresponding to said group based on the data of the patient samples belonging to said group includes:

using the data of the patient samples that belongs to said group in the training set to build a machine learning model by quantifying the patient information of the patient samples that belongs to said group in the training set, and building a machine learning model between the quantified patient information and the actual test results in the training set, where the machine learning model satisfies the following formula:

$$X_t = f(x_{1t}, x_{2t}, \cdots, x_{nt}) + \varepsilon_t$$

where $X_t$ is an actual test result in the training set, $f(x_{1t}, x_{2t}, \cdots, x_{nt})$ is the machine learning model, $x_{1t}, x_{2t}, \cdots, x_{nt}$ represents the quantified patient information, and $\varepsilon_t$ represents a residual estimated by using the machine learning model $X_t$. In other words, a machine learning model $f$ is trained by performing regression learning on historical test results of the testing instrument in a controlled state and the patient information, where $\varepsilon_t$ at this time approximately assumes a random distribution with a mean value of 0. Here, the order of the residual $\varepsilon_t$ sequence still follows the original test time order of the patient samples.

In some embodiments, building, based on the machine learning models corresponding to the plurality of groups, a quality control model that is used for performing real-time quality control based on patient samples, may include: building the quality control model based on the machine learning models corresponding to the plurality of groups and a shared process control SPC algorithm-based computational model, where, differences between the actual test results and test results predicted by the machine learning model $f$, that is, residuals $\varepsilon_t$, are input into the shared process control SPC algorithm-based computational model to obtain outputs of the computational model as monitoring indicators of the quality control model. In other words, differences between the actual test results and the predicted test results for the patient samples of each group, that is, the residuals $\varepsilon_t$, are obtained first based on the machine learning model corresponding to each group, and then the residuals $\varepsilon_t$ of all patient samples are input into the shared process control SPC algorithm-based computational model to obtain monitoring indicators.

However, in other embodiments, the monitoring indicators of the quality control model may be calculated in other ways instead, for example, by calculating ratios between the actual test results and the test results predicted by the machine learning model $f$.

In an embodiment of the disclosure, the method 100 may be implemented in the form of software such as middleware.

In some embodiments, the method 100 may be integrated into a laboratory (clinical laboratory department) information system (LIS for short) or a hospital information system (HIS) in the form of software.

As shown in FIG. 12, an embodiment of the disclosure further provides a sample testing system 800. The sample testing system 800 includes a testing instrument 810, for example a plurality of testing instruments configured to test a test sample for a target item, and a controller 820. The testing instruments include, for example, a hematology analyzer or a biochemical analyzer.

Here, the controller 820 is communicatively connected to the testing instrument 810 and is configured to implement the method 100 or one of implementations of the method.

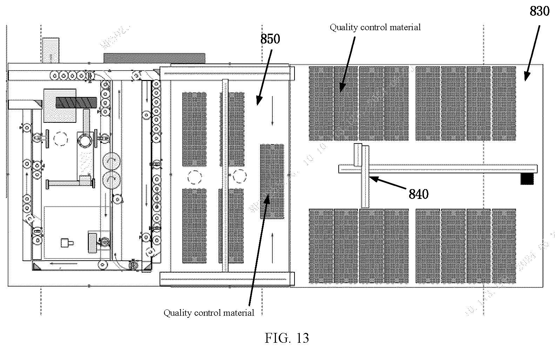

In some embodiments, as shown in FIG. 13, the sample testing system 800 further includes a storage device 830 configured to store a quality control material and a dispatching device 840 configured to retrieve the quality control material. Accordingly, the controller 820 is further configured to:

control, if it is determined that the first alarm prompt is unreliable, the dispatching device 840 to retrieve the quality control material from the storage device 830 and to transfer the quality control material to the testing instrument 810 for testing, so as to obtain a quality control test result of the quality control material for the target item.

In some embodiment, the dispatching device 840 may include a motor and a conveyor belt. In other alternative or additional embodiments, the dispatching device 840 may include manipulator.

In some embodiments, the storage device is a refrigerator device. For example, the refrigerator device includes a storage device configured to store the quality control material and a refrigeration component configured to refrigerate the storage device.

In some embodiments, as shown in FIG. 13, the sample testing system 800 further includes a temporary storage device 850 such as a temporary storage platform. Accordingly, the controller 820 is further configured to, when the dispatching device 840 retrieves the quality control material from the storage device 830 and transfers the quality control material to the testing instrument 810 for testing, perform the following steps:

controlling the dispatching device 840 to retrieve the quality control material from the storage device 830 and to transfer the quality control material to the temporary storage device 850 for temporary storage; and

controlling the dispatching device 840 to retrieve the quality control material from the temporary storage device 850 and to transfer the quality control material to the testing instrument 810 for testing, so as to obtain the quality control test result.

In other embodiments, alternatively, the controller 820 may be configured to: control, after the prompt to perform quality control again by using the quality control material is output, in response to an input operation of a user with respect to the prompt, the testing instrument to test the quality control material for the target item to obtain another quality control test result.

For example, the sample testing system further includes a user interaction device, such as a user interaction interface. Through the user interaction device, the user manually confirms a need to perform IQC and manually sends the quality control material to the testing instrument for testing. The controller 820 receives an instruction from the user interaction device to perform IQC, and in response to the instruction, controls the testing instrument to test the quality control material.

For another example, through the user interaction device, the user manually confirms a need to perform IQC. The controller 820 receives, from the user interaction device, an instruction to perform IQC, and in response to the instruction, automatically dispatches the quality control material to the testing instrument for testing. For example, the controller controls the dispatching device to retrieve the quality control material from the storage device and to transfer the quality control material to the testing instrument for testing, so as to obtain a quality control test result.

In some implementations, the controller 820 may include: one or more processors; and one or more computer-readable memories coupled to the one or more processors, where the computer-readable memories are configured to store a series of computer-executable instructions, and the series of computer-executable instructions, when executed by the one or more processors, cause the processor or processors to perform the corresponding method steps.

In some examples, the processors in an embodiment of the disclosure may include, but are not limited to, a central processing unit, a micro controller unit, a field-programmable gate array, a digital signal processor, and other devices configured to interpret computer instructions and process data in computer software.

In some examples, the memory in an embodiment of the disclosure may be a volatile memory or a non-volatile memory, or may include both a volatile memory and a non-volatile memory. The non-volatile memory may be a read-only memory, a programmable read-only memory, an erasable programmable read-only memory, an electrically erasable programmable read-only memory, a magnetic random-access memory, a flash memory, a magnetic surface memory, an optical disc, or a compact disc read-only memory. The magnetic surface memory may be a disk memory or a magnetic tape memory. The volatile memory may be a random-access memory, and is used as an external cache. By way of example instead of limitation, many forms of RAMs are available, such as a static random access memory, a synchronous static random access memory, a dynamic random access memory, a synchronous dynamic random access memory, a double data rate synchronous dynamic random access memory, an enhanced synchronous dynamic random access memory, a synclink dynamic random access memory, and a direct RAMBUS dynamic random access memory. The memory described in the embodiments of the disclosure is intended to include, but not limited to, these memories and any other suitable types of memories.

Further, the controller 820 may further include a communication interface. The communication interface communicates with the processor and a computer-readable storage medium through a bus. The communication interface may be an interface that supports any currently known communication protocol. The communication interface may communicate with an external device through a network. Through the communication interface, the controller may exchange data, based on a communication protocol, with any device connected to the controller through the communication interface.

For further embodiments of the sample testing system and the advantages thereof, reference may be made to the above description about the methods. In other words, each embodiment of each method, and preferred advantages thereof, are equally applicable to the sample testing system, and will not be described again here.

An embodiment of the disclosure further relates to a device for quality control of a testing instrument, including: a non-transitory computer-readable storage medium with computer-readable instructions stored thereon; and one or more processors, configured to execute the computer-readable instructions to implement the method 100 or an embodiment thereof.

An embodiment of the disclosure further relates to a computer-readable storage medium with a computer program stored thereon, where the computer program. when executed by a processor, implements the method 100 or an embodiment thereof.

A person skilled in the art understands that an embodiment of the disclosure may be provided as a method, a system, or a computer program product. Therefore, an embodiment of the disclosure may be implemented in the form of a hardware embodiment, a software embodiment, or a combination thereof. Moreover, an embodiment of the disclosure may be implemented in the form of a computer program product that is implemented on one or more computer-usable storage media (including a disk memory, an optical memory, and the like) that include computer-usable program code.

The embodiments of the disclosure are described with reference to flowcharts and/or block diagrams of the methods, devices (systems), and computer program products according to the embodiments of the disclosure. It should be understood that each procedure and/or block in the flowcharts and/or block diagrams, and combinations of the procedures and/or blocks in the flowcharts and/or block diagrams may be implemented by computer program operations. These computer program operations may be provided to a processor of a general-purpose computer, a special-purpose computer, an embedded processor or other programmable data processing devices to create a machine, such that the operations executed by the processor of the computer or other programmable data processing devices create a device for implementing functions specified in one or more procedures in the flowcharts and/or one or more blocks in the block diagrams.

These computer program operations may also be stored in a computer-readable memory that may direct a computer or other programmable data processing device to operate in a specific manner, such that the operations stored in the computer-readable memory create a manufacture article including an operation device, and the operation device implements the functions specified in one or more procedures in the flowcharts and/or one or more blocks in the block diagrams.

These computer program operations may also be loaded onto a computer or other programmable data processing devices to enable a series of operation steps to be executed on the computer or other programmable devices to perform computer-implemented processing, such that the operations executed on the computer or other programmable devices provide steps for implementing the functions specified in one or more procedures in the flowcharts and/or one or more blocks in the block diagrams.

The features or combinations thereof mentioned above in the description, accompanying drawings, and claims can be combined with each other arbitrarily or used separately as long as they are meaningful within the scope of the disclosure and do not contradict each other. The advantages and features described with respect to the method provided herein are correspondingly applicable to the system and applications provided herein, and vice versa.

The foregoing description merely relates to the preferred embodiments of the disclosure, and is not intended to limit the scope of patent of the disclosure. All equivalent variations made by using the content of the specification and the accompanying drawings of the disclosure from the concept of the disclosure, or the direct/indirect applications of the contents in other related technical fields all fall within the scope of patent protection of the disclosure.


EXAMPLES

1. A method for quality control of a testing instrument, the method comprising:

obtaining a quality control test result of a quality control material that simulates a biological sample, and obtaining a first monitoring indicator based on the quality control test result, wherein the quality control test result is obtained by the testing instrument testing the quality control material for a target item at a quality control time point; determining whether the testing instrument is out of control based on the first monitoring indicator; when the testing instrument is out of control, outputting a first alarm prompt indicating that the testing instrument is out of control, and obtaining actual test results of a plurality of actual biological samples tested by the testing instrument for the target item, and inputting the actual test results into a patient-based real-time quality control (PBRTQC)-based quality control model to obtain second monitoring indicators, wherein obtaining actual test results of a plurality of actual biological samples tested by the testing instrument for the target item comprises: 1) obtaining the actual test results of the plurality of actual biological samples tested by the testing instrument for the target item within a preset period of time before and/or after the quality control time point, or 2) obtaining actual test results of a preset number of biological samples tested by the testing instrument for the target item before and/or after the quality control time point; and determining whether the first alarm prompt is reliable based on the second monitoring indicators.

2. The method of example 1, wherein obtaining the actual test results of the plurality of actual biological samples tested by the testing instrument for the target item within a preset period of time before and/or after the quality control time point, comprises: obtaining the actual test results of the plurality of actual biological samples tested by the testing instrument for the target item within 24 hours before or after the quality control time point.

3. The method of example 2, wherein obtaining the actual test results of the plurality of actual biological samples tested by the testing instrument for the target item within a preset period of time before and/or after the quality control time point, comprises:

obtaining the actual test results of the plurality of actual biological samples tested by the testing instrument for the target item within 24 hours, preferably 18 hours, before the quality control time point; or
obtaining the actual test results of the plurality of actual biological samples tested by the testing instrument for the target item within 2 hours after the quality control time point.

4. The method of any one of examples 1 to 3, wherein determining whether the first alarm prompt is reliable based on the second monitoring indicators, comprises:
determining that the first alarm prompt is reliable, and preferably outputting a second alarm prompt indicating that the testing instrument is out of control, when it is determined that the testing instrument is out of control based on the second monitoring indicators.

5. The method of any one of examples 1 to 4, wherein determining whether the first alarm prompt is reliable based on the second monitoring indicators, comprises:
determining that the first alarm prompt is unreliable, and outputting a third alarm prompt indicating that the first alarm prompt is unreliable, when it is determined that the testing instrument is in control based on the second monitoring indicators.

6. The method of example 5, further comprising:
outputting prompt information indicating that a possible reason for unreliability of the first alarm prompt lies in the quality control material rather than the testing instrument, when it is determined that the first alarm prompt is unreliable.

7. The method of example 5 or 6, further comprising:
outputting prompt information indicating that the second monitoring indicators should be used as a quality control reference, when it is determined that the first alarm prompt is unreliable.

8. The method of any one of examples 5 to 7, further comprising:
outputting, when it is determined that the first alarm prompt is unreliable, a prompt to perform quality control again by using the quality control material or by using another quality control material, preferably by using another quality control material in a different batch from the quality control material, or, controlling dispatching of the quality control material or another quality control material to the testing instrument, so as to obtain another quality control test result of the quality control material or the another quality control material for the target item.

9. The method of example 8, wherein after outputting a prompt to perform quality control again by using the quality control material or by using another quality control material, the method further comprises:
controlling, in response to an input operation of a user with respect to the prompt, the testing instrument to test the quality control material or the another quality control material for the target item, so as to obtain the another quality control test result.

10. The method of any one of examples 1 to 9, wherein the PBRTQC-based quality control model comprises a statistical process control (SPC) algorithm-based computational model; and
inputting the actual test results into a patient-based real-time quality control (PBRTQC)-based quality control model to obtain second monitoring indicators, comprises: inputting the actual test results into the SPC algorithm-based computational model, and using output results of the computational model as the second monitoring indicators.

11. The method of any one of examples 1 to 10, wherein the PBRTQC-based quality control model comprises a machine learning model.

12. The method of example 11, wherein inputting the actual test results into a patient-based real-time quality control (PBRTQC)-based quality control model to obtain second monitoring indicators, comprises:

obtaining patient information of actual patients corresponding to the plurality of actual biological samples, wherein the patient information comprises at least one of a disease, an age, a gender, and an ordering department of the target item, and preferably the patient information comprises a disease, an age, a gender, and an ordering department of the target item;

quantifying the patient information of actual patients corresponding to the plurality of actual biological samples, and inputting the quantified patient information into the machine learning model, and using output results of the machine learning model as predicted test results for the target item, wherein the machine learning model is obtained by training with actual test results of biological samples of a plurality of historical patients for the target item and patient information of the plurality of historical patients; and

obtaining the second monitoring indicators based on the actual test results and the predicted test results of the plurality of actual biological samples.

13. The method of example 12, wherein obtaining the second monitoring indicators based on the actual test results and the predicted test results of the plurality of actual biological samples, comprises:

calculating differences between the actual test results and the predicted test results of the plurality of actual biological samples; and

inputting the differences into a statistical process control (SPC) algorithm-based computational model, and using output results of the computational model as the second monitoring indicators.

14. The method of example 13, wherein quantifying the patient information of actual patients corresponding to the plurality of actual biological samples comprises:

for each of the actual biological samples, quantifying at least one type of the patient information of the actual patient corresponding to said actual biological sample, especially at least one of the disease, the gender, and the ordering department of the target item, into a matrix.

15. The method of any one of examples 11 to 14, wherein the machine learning model is a neural network model, an SVM -based machine learning model, or an LDA-based machine learning model.

16. The method of any one of examples 1 to 15, wherein inputting the actual test results into a patient-based real-time quality control (PBRTQC)-based quality control model to obtain second monitoring indicators, comprises: for each of the plurality of actual biological samples,

selecting a group matching said actual biological sample from a plurality of groups, and preferably selecting a group matching said actual biological sample from the plurality of groups based on a preset criterion, wherein the plurality of groups are divided based on the preset criterion; and

inputting the actual test result of said actual biological sample into the PBRTQC-based quality control model corresponding to the selected group, so as to obtain the second monitoring indicators.

17. The method of example 16, wherein the preset criterion comprises a plurality of threshold ranges of a test result of the target item, and preferably the preset criterion comprises a reference range or a clinical decision level of a test result of the target item; and

preferably, the plurality of threshold ranges correspond one-to-one to the plurality of groups, and selecting a group matching said actual biological sample from the plurality of groups based on a preset criterion comprises:

selecting, from the plurality of threshold ranges, a threshold range within which the actual test result of said actual biological sample falls, and using a group corresponding to the selected threshold range as the group matching said actual biological sample.

18. The method of examples 1 to 17, further comprising: displaying the first monitoring indicator and the second monitoring indicators on a same display interface.

19. The method of example 18, further comprising:

labeling, on the display interface, the actual biological sample, if it is determined that the testing instrument is out of control based on the first monitoring indicator, and on the second monitoring indicators associated with the actual biological sample.

20. The method of example 18 or 19, wherein displaying the first monitoring indicator and the second monitoring indicators on a same display interface comprises:

displaying the first monitoring indicator in a first coordinate system on the same display interface, wherein an ordinate of the first coordinate system represents the first monitoring indicator and an abscissa of the first coordinate system represents a test time point of the quality control material; and displaying the second monitoring indicators in a second coordinate system on the same display interface, wherein an ordinate of the second coordinate system represents the second monitoring indicators, and an abscissa of the second coordinate system represents a test time point of each of the plurality of actual biological samples, or a serial number of each of the plurality of actual biological samples, or a test order of the plurality of actual biological samples; or

displaying the first monitoring indicator and the second monitoring indicators in a same coordinate system on the same display interface, wherein an ordinate of the same second coordinate system represents the first monitoring indicator and the second monitoring indicators, and an abscissa of the same coordinate system represents a test time point of the quality control material and a test time point of each of the plurality of actual biological samples.

21. A sample testing system, comprising:

a testing instrument configured to test a test sample for a target item; and
a controller communicatively connected to the testing instrument and configured to carry out the method of any one of examples 1 to 20.

22. A device for performing quality control of a testing instrument, the device comprising:

a non-transitory computer-readable storage medium with computer-readable instructions stored thereon; and
one or more processors configured to execute the computer-readable instructions to carry out the method of any one of examples 1 to 20.

23. A computer-readable storage medium with a computer program stored thereon, wherein the computer program, when executed by a processor, implements the method of any one of examples 1 to 20.

**Claims**

1. A method for quality control of a testing instrument, the method comprising:

obtaining a quality control test result of a quality control material that simulates a biological sample, and obtaining a first monitoring indicator based on the quality control test result, wherein the quality control test result is obtained by the testing instrument testing the quality control material for a target item at a quality control time point;
determining whether the testing instrument is out of control based on the first monitoring indicator; when the testing instrument is out of control, outputting a first alarm prompt indicating that the testing instrument is out of control, and obtaining actual test results of a plurality of actual biological samples tested by the testing instrument for the target item, and inputting the actual test results into a patient-based real-time quality control (PBRTQC)-based quality control model to obtain second monitoring indicators, wherein obtaining actual test results of a plurality of actual biological samples tested by the testing instrument for the target item comprises: 1) obtaining the actual test results of the plurality of actual biological samples tested by the testing instrument for the target item within a preset period of time before and/or after the quality control time point, or 2) obtaining actual test results of a preset number of biological samples tested by the testing instrument for the target item before and/or after the quality control time point; and
determining whether the first alarm prompt is reliable based on the second monitoring indicators.

2. The method of claim 1, wherein obtaining the actual test results of the plurality of actual biological samples tested by the testing instrument for the target item within a preset period of time before and/or after the quality control time point, comprises:
obtaining the actual test results of the plurality of actual biological samples tested by the testing instrument for the target item within 24 hours before or after the quality control time point.

3. The method of claim 2, wherein obtaining the actual test results of the plurality of actual biological samples tested by the testing instrument for the target item within a preset period of time before and/or after the quality control time point, comprises:

obtaining the actual test results of the plurality of actual biological samples tested by the testing instrument for the target item within 24 hours, preferably 18 hours, before the quality control time point; or
obtaining the actual test results of the plurality of actual biological samples tested by the testing instrument for the target item within 2 hours after the quality control time point.

4. The method of any one of claims 1 to 3, wherein determining whether the first alarm prompt is reliable based on the second monitoring indicators, comprises:
determining that the first alarm prompt is reliable, and preferably outputting a second alarm prompt indicating that the testing instrument is out of control, when it is determined that the testing instrument is out of control based on the second monitoring indicators.

5. The method of any one of claims 1 to 4, wherein determining whether the first alarm prompt is reliable based on the second monitoring indicators, comprises:

determining that the first alarm prompt is unreliable, and outputting a third alarm prompt indicating that the first alarm prompt is unreliable, when it is determined that the testing instrument is in control based on the second monitoring indicators;
optionally the method further comprises:

outputting prompt information indicating that a possible reason for unreliability of the first alarm prompt lies in the quality control material rather than the testing instrument, when it is determined that the first alarm prompt is unreliable;
optionally, the method further comprises:
outputting prompt information indicating that the second monitoring indicators should be used as a quality control reference, when it is determined that the first alarm prompt is unreliable.

6. The method of claim 5, further comprising:

outputting, when it is determined that the first alarm prompt is unreliable, a prompt to perform quality control again by using the quality control material or by using another quality control material, preferably by using another quality control material in a different batch from the quality control material, or, controlling dispatching of the quality control material or another quality control material to the testing instrument, so as to obtain another quality control test result of the quality control material or the another quality control material for the target item.
optionally, after outputting a prompt to perform quality control again by using the quality control material or by using another quality control material, the method further comprises:
controlling, in response to an input operation of a user with respect to the prompt, the testing instrument to test the quality control material or the another quality control material for the target item, so as to obtain the another quality control test result.

7. The method of any one of claims 1 to 6, wherein the PBRTQC-based quality control model comprises a machine learning model;
optionally, the machine learning model is a neural network model, an SVM -based machine learning model, or an LDA-based machine learning model.

8. The method of claim 7, wherein inputting the actual test results into a patient-based real-time quality control (PBRTQC)-based quality control model to obtain second monitoring indicators, comprises:

obtaining patient information of actual patients corresponding to the plurality of actual biological samples, wherein the patient information comprises at least one of a disease, an age, a gender, and an ordering department of the target item, and preferably the patient information comprises a disease, an age, a gender, and an ordering department of the target item;
quantifying the patient information of actual patients corresponding to the plurality of actual biological samples, and inputting the quantified patient information into the machine learning model, and using output results of the machine learning model as predicted test results for the target item, wherein the machine learning model is obtained by training with actual test results of biological samples of a plurality of historical patients for the target item and patient information of the plurality of historical patients; and
obtaining the second monitoring indicators based on the actual test results and the predicted test results of the plurality of actual biological samples.

9. The method of claim 8, wherein obtaining the second monitoring indicators based on the actual test results and the predicted test results of the plurality of actual biological samples, comprises:

calculating differences between the actual test results and the predicted test results of the plurality of actual biological samples; and

inputting the differences into a statistical process control (SPC) algorithm-based computational model, and using output results of the computational model as the second monitoring indicators.

10. The method of any one of claims 1 to 9, wherein inputting the actual test results into a patient-based real-time quality control (PBRTQC)-based quality control model to obtain second monitoring indicators, comprises: for each of the plurality of actual biological samples,

selecting a group matching said actual biological sample from a plurality of groups, and preferably selecting a group matching said actual biological sample from the plurality of groups based on a preset criterion, wherein the plurality of groups are divided based on the preset criterion; and

inputting the actual test result of said actual biological sample into the PBRTQC-based quality control model corresponding to the selected group, so as to obtain the second monitoring indicators.

11. The method of claim 10, wherein the preset criterion comprises a plurality of threshold ranges of a test result of the target item, and preferably the preset criterion comprises a reference range or a clinical decision level of a test result of the target item; and

preferably, the plurality of threshold ranges correspond one-to-one to the plurality of groups, and selecting a group matching said actual biological sample from the plurality of groups based on a preset criterion comprises:

selecting, from the plurality of threshold ranges, a threshold range within which the actual test result of said actual biological sample falls, and using a group corresponding to the selected threshold range as the group matching said actual biological sample.

12. The method of claims 1 to 11, further comprising: displaying the first monitoring indicator and the second monitoring indicators on a same display interface.

13. The method of claim 12, further comprising:

labeling, on the display interface, the actual biological sample, if it is determined that the testing instrument is out of control based on the first monitoring indicator, and on the second monitoring indicators associated with the actual biological sample.

14. The method of claim 12 or 13, wherein displaying the first monitoring indicator and the second monitoring indicators on a same display interface comprises:

displaying the first monitoring indicator in a first coordinate system on the same display interface, wherein an ordinate of the first coordinate system represents the first monitoring indicator and an abscissa of the first coordinate system represents a test time point of the quality control material; and displaying the second monitoring indicators in a second coordinate system on the same display interface, wherein an ordinate of the second coordinate system represents the second monitoring indicators, and an abscissa of the second coordinate system represents a test time point of each of the plurality of actual biological samples, or a serial number of each of the plurality of actual biological samples, or a test order of the plurality of actual biological samples; or

displaying the first monitoring indicator and the second monitoring indicators in a same coordinate system on the same display interface, wherein an ordinate of the same second coordinate system represents the first monitoring indicator and the second monitoring indicators, and an abscissa of the same coordinate system represents a test time point of the quality control material and a test time point of each of the plurality of actual biological samples.

15. A sample testing system, comprising:

a testing instrument configured to test a test sample for a target item; and

a controller communicatively connected to the testing instrument and configured to carry out the method of any one of claims 1 to 14.

100

S110

| Obtaining a first monitoring indicator based on IQC |

S120

| Determining whether the testing instrument is out of control based on the first monitoring indicator; and outputting a first alarm prompt indicating that the testing instrument is out of control, and obtaining second monitoring indicators based on PBRTQC, if the testing instrument is out of control |

S130

| Determining whether the first alarm prompt is reliable based on the second monitoring indicators |

FIG. 1

FIG. 2

FIG. 3

FIG. 4

FIG. 5

300

S310

Obtaining data of a plurality of patient samples and dividing the data into a training set and a test set

S320

Using the training set to building a machine learning model

S330

Building a quality control model based on the machine learning model

S340

Verifying the quality control model by using the test set

FIG. 6

330

S331a

Setting a false alarm rate

S332a

Setting a plurality of SPC parameter values of the computational model

S333a

Determining an upper control limit, a lower control limit, and an ANPed at the false alarm rate for each SPC parameter value

S334a

Selecting an SPC parameter value with a minimum ANPed and the upper and lower control limits corresponding to the SPC parameter value

FIG. 7

330

S331b
Setting a false alarm rate

S332b
Setting a plurality of truncation percentages for the truncation process and a plurality of SPC parameter values of the computational model

S333b
Determining an upper control limit, a lower control limit, and an ANPed at the false alarm rate for each parameter value combination

S334b
Selecting a parameter value combination with a minimum ANPed and the upper and lower control limits corresponding to the parameter value combination

FIG. 8

330

S331c
Setting a false alarm rate

S332c
Setting a plurality of truncation percentages for the truncation process, a plurality of values of M, a plurality of values of N, and a plurality of SPC parameter values of the computational model

S333c
Determining an upper control limit, a lower control limit, and an ANPed at the false alarm rate for each parameter value combination

S334c
Selecting a parameter value combination with a minimum ANPed and the upper and lower control limits corresponding to the parameter value combination

FIG. 9

FIG. 10

FIG. 11

FIG. 12

FIG. 13

**EUROPEAN SEARCH REPORT**

Application Number

EP 25 18 6460

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 117 129 698 A (BEIJING ZONGCI TECH DEVELOPMENT CO LTD) 28 November 2023 (2023-11-28) * paragraphs [0008], [0026]; figure 2 * ----- | 1-15 | INV. G01N35/00 |
| A | DUAN XINCEN ET AL: "Assessment of patient-based real-time quality control algorithm performance on different types of analytical error", CLINICA CHIMICA ACTA, vol. 511, 1 December 2020 (2020-12-01), pages 329-335, XP093327616, AMSTERDAM, NL ISSN: 0009-8981, DOI: 10.1016/j.cca.2020.10.006 * the whole document * ----- | 1-15 | |
| A | WANG JINGYUAN ET AL: "Integrating Patient-Based Real-Time Quality Control (PBRTQC) in a New Field: Inter-Comparison between Biochemical Instrumentations with LDL-C", DIAGNOSTICS, vol. 14, no. 9, 23 April 2024 (2024-04-23), page 872, XP093327614, CH ISSN: 2075-4418, DOI: 10.3390/diagnostics14090872 * the whole document * ----- | 1-15 | TECHNICAL FIELDS SEARCHED (IPC) G01N |

The present search report has been drawn up for all claims

| Place of search | Date of completion of the search | Examiner |
|---|---|---|
| The Hague | 21 October 2025 | Böhler, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 6460

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2025

| Patent document cited in search report | Publication date | Patent family member(s) | Publication date |
|---|---|---|---|
| CN 117129698 A | 28-11-2023 | NONE | |

EPO FORM P0459

For more details about this annex : see Official Journal of the European Patent Office, No. 12/82